# FASCICULE DE BREVET EUROPEEN

(11) **EP 2 132 305 B1**
(45) Date de publication et mention de la délivrance du brevet: **15.05.2013**
(21) Numéro de dépôt: 08788102.5
(22) Date de dépôt: 02.04.2008
(51) Int. Cl.: C12N 9/10, C12N 15/52

(54) **GENES CODANT POUR LA Z,Z-FARNESYL DIPHOSPHATE SYNTHASE ET UNE SESQUITERPENE SYNTHASE A PRODUITS MULTIPLES ET LEURS UTILISATIONS**
GENE ZUR KODIERUNG VON Z,Z-FARNESYL-DIPHOSPHAT-SYNTHASE UND EINER SESQUITERPENSYNTHASE MIT MEHREREN PRODUKTEN SOWIE ANWENDUNGEN DAVON
GENES ENCODING Z,Z-FARNESYL DIPHOSPHATE SYNTHASE AND A SESQUITERPENE SYNTHASE WITH MULTIPLE PRODUCTS AND USES THEREOF

(30) Priorité: 03.04.2007 FR 0754235
(43) Date de publication de la demande: 16.12.2009
(73) Titulaire: Philip Morris Products S.A., 2000 Neuchâtel (CH)
(72) Inventeur: SALLAUD, Christophe, F-34070 Montpellier (FR); RONTEIN, Denis, F-04800 Gréoux Les Bains (FR); TISSIER, Alain, F-84120 Pertuis (FR)
(74) Mandataire: Gallois, Valérie
(86) Numéro de dépôt international: PCT/FR2008/050576
(87) Numéro de publication internationale: WO 2008/142318

(56) Documents cités:
- WO-A-2004/111183
- DATABASE EMBL [Online] 27 mars 2000 (2000-03-27), "EST323045 L. hirsutum trichome, Cornell University Lycopersicon hirsutum cDNA clone cLHT11N12 5', mRNA sequence." XP002459624 extrait de EBI accession no. EMBL:AW616634 Database accession no. AW616634
- ADAMS S R ET AL: "EVIDENCE FOR TRANS TRANS FARNESYL PYRO PHOSPHATE AND CIS CIS FARNESYL PYRO PHOSPHATE SYNTHESIS IN GOSSYPIUM-HIRSUTUM" PHYTOCHEMISTRY (OXFORD), vol. 12, no. 9, 1973, pages 2167-2172, XP002459621 ISSN: 0031-9422
- HEINSTEIN P F ET AL: "BIOSYNTHESIS OF GOSSYPOL INCORPORATION OF MEVALONATE 2 CARBON-14 AND ISOPRENYL PYRO PHOSPHATES" JOURNAL OF BIOLOGICAL CHEMISTRY, vol. 245, no. 18, 1970, pages 4658-4665, XP002459622 ISSN: 0021-9258
- KOLLNER ET AL: "Two pockets in the active site of maize sesquiterpene synthase TPS4 carry out sequential parts of the reaction scheme resulting in multiple products" ARCHIVES OF BIOCHEMISTRY AND BIOPHYSICS, NEW YORK, US, US, vol. 448, no. 1-2, 15 avril 2006 (2006-04-15), pages 83-92, XP005414689 ISSN: 0003-9861
- VAN DER HOEVEN R S ET AL: "Genetic control and evolution of sesquiterpene biosynthesis in Lycopersicon esculentum and L. hirsutum" GENBANK, 21 décembre 2000 (2000-12-21), XP002992844

### SEQUENCE LISTING

<110> LIBROPHYT
<120> GENES CODANT POUR LA Z,Z-FARNESYL DIPHOSPHATE SYNTHASE ET UNE SESQUITERPENE SYNTHASE A PRODUITS MULTIPLES ET LEURS UTILISATIONS
<130> B0S63WO
<160> 10
<170> PatentIn version 3.3
<210> 1
   <211> 912
   <212> DNA
   <213> Solanum abutiloides
<220>
   <221> CDS
   <222> (1)..(912)
<400> 1
   atg agt tct ttg gtt ctt caa tgt tgg aaa tta tca tct cca tct ctg      48
   Met Ser Ser Leu Val Leu Gln Cys Trp Lys Leu Ser Ser Pro Ser Leu
   1               5                   10                  15
   att tta caa caa aat aca tca ata tcc atg ggt gca ttc aaa ggt att      96
   Ile Leu Gln Gln Asn Thr Ser Ile Ser Met Gly Ala Phe Lys Gly Ile
               20                  25                  30
   cat aaa ctt caa atc cca aat tca cct ctg aca gtg tct gct cgt gga      144
   His Lys Leu Gln Ile Pro Asn Ser Pro Leu Thr Val Ser Ala Arg Gly
           35                  40                  45
   ctc aac aag att tca tgc tca ctc agc tta caa acc gaa aaa ctt tgt      192
   Leu Asn Lys Ile Ser Cys Ser Leu Ser Leu Gln Thr Glu Lys Leu Cys
       50                  55                  60
   tat gag gat aat gat aat gat ctt gat gaa gaa ctt atg cct aaa cac      240
   Tyr Glu Asp Asn Asp Asn Asp Leu Asp Glu Glu Leu Met Pro Lys His
   65                  70                  75                  80
   att gct ttg ata atg gat ggt aat agg aga tgg gca aag gat aag ggt      288
   Ile Ala Leu Ile Met Asp Gly Asn Arg Arg Trp Ala Lys Asp Lys Gly
                   85                  90                  95
   tta gac gta tcc gaa ggt cac aaa cat ctc ttt cca aaa tta aaa gag      336
   Leu Asp Val Ser Glu Gly His Lys His Leu Phe Pro Lys Leu Lys Glu
               100                 105                 110
   att tgt gac att tct tct aaa ttg gga ata caa gtt atc act gct ttt      384
   Ile Cys Asp Ile Ser Ser Lys Leu Gly Ile Gln Val Ile Thr Ala Phe
           115                 120                 125
   gca ttc tct act gaa aat tgg aaa cga gcc aag ggg gag gtt gat ttc      432
   Ala Phe Ser Thr Glu Asn Trp Lys Arg Ala Lys Gly Glu Val Asp Phe
       130                 135                 140
   ttg atg caa atg ttc gaa gaa ctc tat gat gag ttt tcg agg tct gga      480
   Leu Met Gln Met Phe Glu Glu Leu Tyr Asp Glu Phe Ser Arg Ser Gly
   145                 150                 155                 160
   gta aga gtg tct att att ggt tgt aaa acc gac ctc cca atg aca tta      528
   Val Arg Val Ser Ile Ile Gly Cys Lys Thr Asp Leu Pro Met Thr Leu
                   165                 170                 175
   caa aaa tgc ata gca tta aca gaa gag act aca aag gga aac aaa gga      576
   Gln Lys Cys Ile Ala Leu Thr Glu Glu Thr Thr Lys Gly Asn Lys Gly
               180                 185                 190
   ctt cac ctt gtg att gca cta aac tat ggt ggc tat tat gac ata ttg        624
   Leu His Leu Val Ile Ala Leu Asn Tyr Gly Gly Tyr Tyr Asp Ile Leu
           195                 200                 205
   caa gca aca aaa agc att gtt aat aaa gca atg aat ggt tta tta gat        672
   Gln Ala Thr Lys Ser Ile Val Asn Lys Ala Met Asn Gly Leu Leu Asp
       210                 215                 220
   gta gaa gat atc aac aag aat tta ttt gat caa gaa ctt gaa agc aag        720
   Val Glu Asp Ile Asn Lys Asn Leu Phe Asp Gln Glu Leu Glu Ser Lys
   225                 230                 235                 240
   tgt cca aat cct gat tta ctt ata agg aca gga ggt gat caa aga gtt        768
   Cys Pro Asn Pro Asp Leu Leu Ile Arg Thr Gly Gly Asp Gln Arg Val
                   245                 250                 255
   agt aac ttt ttg ttg tgg caa ttg gct tat act gaa ttt tac ttc acc        816
   Ser Asn Phe Leu Leu Trp Gln Leu Ala Tyr Thr Glu Phe Tyr Phe Thr
               260                 265                 270
   aaa aca ttg ttt cct gat ttt gga gag gaa gat ctt aaa gag gca ata        864
   Lys Thr Leu Phe Pro Asp Phe Gly Glu Glu Asp Leu Lys Glu Ala Ile
           275                 280                 285
   ata aac ttt caa caa agg cat aga cgt ttt ggt gga cac aca tat tga        912
   Ile Asn Phe Gln Gln Arg His Arg Arg Phe Gly Gly His Thr Tyr
       290                 295                 300
<210> 2
   <211> 303
   <212> PRT
   <213> Solanum abutiloides
<400> 2
   Met Ser Ser Leu Val Leu Gln Cys Trp Lys Leu Ser Ser Pro Ser Leu
   1               5                   10                  15
   Ile Leu Gln Gln Asn Thr Ser Ile Ser Met Gly Ala Phe Lys Gly Ile
               20                  25                  30
   His Lys Leu Gln Ile Pro Asn Ser Pro Leu Thr Val Ser Ala Arg Gly
           35                  40                  45
   Leu Asn Lys Ile Ser Cys Ser Leu Ser Leu Gln Thr Glu Lys Leu Cys
       50                  55                  60
   Tyr Glu Asp Asn Asp Asn Asp Leu Asp Glu Glu Leu Met Pro Lys His
   65                  70                  75                  80
   Ile Ala Leu Ile Met Asp Gly Asn Arg Arg Trp Ala Lys Asp Lys Gly
                   85                  90                  95
   Leu Asp Val Ser Glu Gly His Lys His Leu Phe Pro Lys Leu Lys Glu
               100                 105                 110
   Ile Cys Asp Ile Ser Ser Lys Leu Gly Ile Gln Val Ile Thr Ala Phe
           115                 120                 125
   Ala Phe Ser Thr Glu Asn Trp Lys Arg Ala Lys Gly Glu Val Asp Phe
       130                 135                 140
   Leu Met Gln Met Phe Glu Glu Leu Tyr Asp Glu Phe Ser Arg Ser Gly
   145                 150                 155                 160
   Val Arg Val Ser Ile Ile Gly Cys Lys Thr Asp Leu Pro Met Thr Leu
                   165                 170                 175
   Gln Lys Cys Ile Ala Leu Thr Glu Glu Thr Thr Lys Gly Asn Lys Gly
               180                 185                 190
   Leu His Leu Val Ile Ala Leu Asn Tyr Gly Gly Tyr Tyr Asp Ile Leu
           195                 200                 205
   Gln Ala Thr Lys Ser Ile Val Asn Lys Ala Met Asn Gly Leu Leu Asp
       210                 215                 220
   Val Glu Asp Ile Asn Lys Asn Leu Phe Asp Gln Glu Leu Glu Ser Lys
   225                 230                 235                 240
   Cys Pro Asn Pro Asp Leu Leu Ile Arg Thr Gly Gly Asp Gln Arg Val
                   245                 250                 255
   Ser Asn Phe Leu Leu Trp Gln Leu Ala Tyr Thr Glu Phe Tyr Phe Thr
               260                 265                 270
   Lys Thr Leu Phe Pro Asp Phe Gly Glu Glu Asp Leu Lys Glu Ala Ile
           275                 280                 285
   Ile Asn Phe Gln Gln Arg His Arg Arg Phe Gly Gly His Thr Tyr
       290                 295                 300
<210> 3
   <211> 2334
   <212> DNA
   <213> Solanum habrochaites
<220>
   <221> CDS
   <222> (1)..(2334)
<400> 3
   atg ata gtt ggc tat aga agc aca atc ata acc ctt tct cat cct aag        48
   Met Ile Val Gly Tyr Arg Ser Thr Ile Ile Thr Leu Ser His Pro Lys
   1               5                   10                  15
   cta ggc aat ggg aaa aca att tca tcc aat gca att ttc cag aga tca        96
   Leu Gly Asn Gly Lys Thr Ile Ser Ser Asn Ala Ile Phe Gln Arg Ser
               20                  25                  30
   tgt aga gta aga tgc agc cac agt acc cct tca tca atg aat ggt ttc       144
   Cys Arg Val Arg Cys Ser His Ser Thr Pro Ser Ser Met Asn Gly Phe
           35                  40                  45
   gaa gat gca agg gat aga ata agg gaa agt ttt ggg aaa gta gag tta      192
   Glu Asp Ala Arg Asp Arg Ile Arg Glu Ser Phe Gly Lys Val Glu Leu
       50                  55                  60
   tct cct tct tcc tat gac aca gca tgg gta gct atg gtc cct tca aaa      240
   Ser Pro Ser Ser Tyr Asp Thr Ala Trp Val Ala Met Val Pro Ser Lys
   65                  70                  75                  80
   cat tca cta aat gag cca tgt ttt cca caa tgt ttg gat tgg att att      288
   His Ser Leu Asn Glu Pro Cys Phe Pro Gln Cys Leu Asp Trp Ile Ile
                   85                  90                  95
   gaa aat caa aga gaa gat gga tct tgg gga cta aac cct agc cat cca      336
   Glu Asn Gln Arg Glu Asp Gly Ser Trp Gly Leu Asn Pro Ser His Pro
               100                 105                 110
   ttg ctt ctc aag gac tca ctt tct tcc act ctt gca tgt ttg ctt gca      384
   Leu Leu Leu Lys Asp Ser Leu Ser Ser Thr Leu Ala Cys Leu Leu Ala
           115                 120                 125
   cta acc aaa tgg aga gtt gga gat gag caa atc aaa aga ggc ctt ggc      432
   Leu Thr Lys Trp Arg Val Gly Asp Glu Gln Ile Lys Arg Gly Leu Gly
       130                 135                 140
   ttt att gaa acc cag agt tgg gca att gat aac aag gat caa att tca      480
   Phe Ile Glu Thr Gln Ser Trp Ala Ile Asp Asn Lys Asp Gln Ile Ser
   145                 150                 155                 160
   cct cta gga ttt gaa att ata ttt ccc agt atg atc aag tct gca gaa      528
   Pro Leu Gly Phe Glu Ile Ile Phe Pro Ser Met Ile Lys Ser Ala Glu
                   165                 170                 175
   aaa cta aac tta aat cta gca att aac aaa aga gat tca aca att aaa      576
   Lys Leu Asn Leu Asn Leu Ala Ile Asn Lys Arg Asp Ser Thr Ile Lys
               180                 185                 190
   aga gca tta cag aat gag ttc acg agg aat att gaa tat atg agt gaa      624
   Arg Ala Leu Gln Asn Glu Phe Thr Arg Asn Ile Glu Tyr Met Ser Glu
           195                 200                 205
   gga gtt ggt gaa tta tgt gat tgg aag gaa ata ata aag tta cat caa      672
   Gly Val Gly Glu Leu Cys Asp Trp Lys Glu Ile Ile Lys Leu His Gln
       210                 215                 220
   agg caa aat ggt tca tta ttt gat tca cca gcc act act gca gct gcc      720
   Arg Gln Asn Gly Ser Leu Phe Asp Ser Pro Ala Thr Thr Ala Ala Ala
   225                 230                 235                 240
   ttg att tac cat cag cat gat aaa aaa tgc tat gaa tat ctt aat tca      768
   Leu Ile Tyr His Gln His Asp Lys Lys Cys Tyr Glu Tyr Leu Asn Ser
                   245                 250                 255
   atc ttg caa caa cac aaa aat tgg gtt ccc act atg tat cca aca aag      816
   Ile Leu Gln Gln His Lys Asn Trp Val Pro Thr Met Tyr Pro Thr Lys
               260                 265                 270
   ata cat tca ttg ctt tgc ttg gtt gat aca ctt caa aat ctt gga gta      864
   Ile His Ser Leu Leu Cys Leu Val Asp Thr Leu Gln Asn Leu Gly Val
           275                 280                 285
   cat cgg cat ttt aaa tca gaa ata aag aaa gct cta gat gaa ata tac      912
   His Arg His Phe Lys Ser Glu Ile Lys Lys Ala Leu Asp Glu Ile Tyr
       290                 295                 300
   agg cta tgg caa caa aag aat gaa caa att ttc tca aat gtc acc cat      960
   Arg Leu Trp Gln Gln Lys Asn Glu Gln Ile Phe Ser Asn Val Thr His
   305                 310                 315                 320
   tgt gct atg gct ttt cga ctt cta agg atg agc tac tat gat gtc tcc      1008
   Cys Ala Met Ala Phe Arg Leu Leu Arg Met Ser Tyr Tyr Asp Val Ser
   325 330 335
   tca gat gaa cta gca gaa ttt gtg gat gaa gaa cat ttt ttt gca ata 1056
   Ser Asp Glu Leu Ala Glu Phe Val Asp Glu Glu His Phe Phe Ala Ile
   340 345 350
   agt ggg aaa tat aca agt cat gtt gaa att ctt gaa ctc cac aaa gca 1104
   Ser Gly Lys Tyr Thr Ser His Val Glu Ile Leu Glu Leu His Lys Ala
   355 360 365
   tca caa ttg gct att gat cat gag aaa gat gac att ttg gat aag att 1152
   Ser Gln Leu Ala Ile Asp His Glu Lys Asp Asp Ile Leu Asp Lys Ile
   370 375 380
   aac aat tgg aca aga aca ttt atg gag caa aaa ctc tta aac aat ggc 1200
   Asn Asn Trp Thr Arg Thr Phe Met Glu Gln Lys Leu Leu Asn Asn Gly
   385 390 395 400
   ttc ata gat agg atg tca aaa aag gag gtg gaa ctt gct ttg agg aag 1248
   Phe Ile Asp Arg Met Ser Lys Lys Glu Val Glu Leu Ala Leu Arg Lys
   405 410 415
   ttt tat acc ata tct gat cta gca gaa aat aga aga tgt ata aag tca 1296
   Phe Tyr Thr Ile Ser Asp Leu Ala Glu Asn Arg Arg Cys Ile Lys Ser
   420 425 430
   tac gaa gag aac aat ttt aaa atc tta aaa gca gct tat agg tca cct 1344
   Tyr Glu Glu Asn Asn Phe Lys Ile Leu Lys Ala Ala Tyr Arg Ser Pro
   435 440 445
   aac att tac aat aag gac ttg ttt ata ttt tca ata cgc aac ttt gaa 1392
   Asn Ile Tyr Asn Lys Asp Leu Phe Ile Phe Ser Ile Arg Asn Phe Glu
   450 455 460
   tta tgc caa gct caa cac caa gaa gaa ctt caa caa ttc aag agg tgg 1440
   Leu Cys Gln Ala Gln His Gln Glu Glu Leu Gln Gln Phe Lys Arg Trp
   465 470 475 480
   ttt gaa gat tat aga ttg gac caa ctc gga att gca gaa cga tat ata 1488
   Phe Glu Asp Tyr Arg Leu Asp Gln Leu Gly Ile Ala Glu Arg Tyr Ile
   485 490 495
   cat gat act tac tta tgt gct gtt att gtt gtc ccc gag cct gaa tta 1536
   His Asp Thr Tyr Leu Cys Ala Val Ile Val Val Pro Glu Pro Glu Leu
   500 505 510
   tcc gat gct cgt ctc ttg tac gcg aaa tac gtc ttg ctc ctg act att 1584
   Ser Asp Ala Arg Leu Leu Tyr Ala Lys Tyr Val Leu Leu Leu Thr Ile
   515 520 525
   gtc gat gat cag ttc gac agt ttt gca tct aca gat gaa tgt ctc aac 1632
   Val Asp Asp Gln Phe Asp Ser Phe Ala Ser Thr Asp Glu Cys Leu Asn
   530 535 540
   atc att gaa tta gta gaa agg tgg gat gac tat gca agt gta ggt tat 1680
   Ile Ile Glu Leu Val Glu Arg Trp Asp Asp Tyr Ala Ser Val Gly Tyr
   545 550 555 560
   aaa tct gag aag gtt aaa gtt ttc ttt tca act ttg tac aaa tca ata 1728
   Lys Ser Glu Lys Val Lys Val Phe Phe Ser Thr Leu Tyr Lys Ser Ile
   565 570 575
   gag gag ctt gta aca att gct gaa att aaa caa gga cga tct gtc aaa 1776
   Glu Glu Leu Val Thr Ile Ala Glu Ile Lys Gln Gly Arg Ser Val Lys
   580 585 590
   aat cac ctt ctt aat ttg tgg ctt gaa ttg gtg aag ttg atg ttg atg 1824
   Asn His Leu Leu Asn Leu Trp Leu Glu Leu Val Lys Leu Met Leu Met
           595                     600                     605
   gaa cga gta gag tgg ttt tct ggc aag aca atc cca agc ata gaa gag      1872
   Glu Arg Val Glu Trp Phe Ser Gly Lys Thr Ile Pro Ser Ile Glu Glu
       610                     615                     620
   tat ttg tat gtt aca tct ata aca ttt ggt gca aga ttg att cct ctc      1920
   Tyr Leu Tyr Val Thr Ser Ile Thr Phe Gly Ala Arg Leu Ile Pro Leu
   625                 630                     635                     640
   aca aca caa tat ttt ctt gga ata aaa ata tcc gaa gat att tta gaa      1968
   Thr Thr Gln Tyr Phe Leu Gly Ile Lys Ile Ser Glu Asp Ile Leu Glu
                   645                     650                     655
   agt gat gaa ata tat ggt tta tgc aac tgt acc ggt aga gtc ctt cga      2016
   Ser Asp Glu Ile Tyr Gly Leu Cys Asn Cys Thr Gly Arg Val Leu Arg
               660                     665                     670
   atc ctt aat gat tta caa gat tcc aag aaa gaa caa aag gag gac tca      2064
   Ile Leu Asn Asp Leu Gln Asp Ser Lys Lys Glu Gln Lys Glu Asp Ser
           675                     680                     685
   gta act ata gtc aca tta cta atg aaa agt atg tct gag gaa gaa gct      2112
   Val Thr Ile Val Thr Leu Leu Met Lys Ser Met Ser Glu Glu Glu Ala
       690                     695                     700
   ata atg aag ata aag gaa atc ttg gaa atg aat aga aga gag tta ttg      2160
   Ile Met Lys Ile Lys Glu Ile Leu Glu Met Asn Arg Arg Glu Leu Leu
   705                 710                     715                     720
   aaa atg gtt tta gtt caa aaa aag gga agc caa ttg cct caa ata tgc      2208
   Lys Met Val Leu Val Gln Lys Lys Gly Ser Gln Leu Pro Gln Ile Cys
                   725                     730                     735
   aaa gat ata ttt tgg agg aca agc aac tgg gct gat ttc att tat tta      2256
   Lys Asp Ile Phe Trp Arg Thr Ser Asn Trp Ala Asp Phe Ile Tyr Leu
               740                     745                     750
   caa act gat gga tat aga att gca gag gaa atg aag aat cac att gat      2304
   Gln Thr Asp Gly Tyr Arg Ile Ala Glu Glu Met Lys Asn His Ile Asp
           755                     760                     765
   gaa gtc ttt tac aaa cca ctc aat cat taa                              2334
   Glu Val Phe Tyr Lys Pro Leu Asn His
       770                     775
<210> 4
   <211> 777
   <212> PRT
   <213> Solanum habrochaites
<400> 4
   Met Ile Val Gly Tyr Arg Ser Thr Ile Ile Thr Leu Ser His Pro Lys
   1               5                   10                  15
   Leu Gly Asn Gly Lys Thr Ile Ser Ser Asn Ala Ile Phe Gln Arg Ser
               20                  25                  30
   Cys Arg Val Arg Cys Ser His Ser Thr Pro Ser Ser Met Asn Gly Phe
           35                  40                  45
   Glu Asp Ala Arg Asp Arg Ile Arg Glu Ser Phe Gly Lys Val Glu Leu
       50                  55                  60
   Ser Pro Ser Ser Tyr Asp Thr Ala Trp Val Ala Met Val Pro Ser Lys
   65                  70                  75                  80
   His Ser Leu Asn Glu Pro Cys Phe Pro Gln Cys Leu Asp Trp Ile Ile
                   85                  90                  95
   Glu Asn Gln Arg Glu Asp Gly Ser Trp Gly Leu Asn Pro Ser His Pro
               100                 105                 110
   Leu Leu Leu Lys Asp Ser Leu Ser Ser Thr Leu Ala Cys Leu Leu Ala
           115                 120                 125
   Leu Thr Lys Trp Arg Val Gly Asp Glu Gln Ile Lys Arg Gly Leu Gly
       130                 135                 140
   Phe Ile Glu Thr Gln Ser Trp Ala Ile Asp Asn Lys Asp Gln Ile Ser
   145                 150                 155                 160
   Pro Leu Gly Phe Glu Ile Ile Phe Pro Ser Met Ile Lys Ser Ala Glu
                   165                 170                 175
   Lys Leu Asn Leu Asn Leu Ala Ile Asn Lys Arg Asp Ser Thr Ile Lys
               180                 185                 190
   Arg Ala Leu Gln Asn Glu Phe Thr Arg Asn Ile Glu Tyr Met Ser Glu
           195                 200                 205
   Gly Val Gly Glu Leu Cys Asp Trp Lys Glu Ile Ile Lys Leu His Gln
       210                 215                 220
   Arg Gln Asn Gly Ser Leu Phe Asp Ser Pro Ala Thr Thr Ala Ala Ala
   225                 230                 235                 240
   Leu Ile Tyr His Gln His Asp Lys Lys Cys Tyr Glu Tyr Leu Asn Ser
                   245                 250                 255
   Ile Leu Gln Gln His Lys Asn Trp Val Pro Thr Met Tyr Pro Thr Lys
               260                 265                 270
   Ile His Ser Leu Leu Cys Leu Val Asp Thr Leu Gln Asn Leu Gly Val
           275                 280                 285
   His Arg His Phe Lys Ser Glu Ile Lys Lys Ala Leu Asp Glu Ile Tyr
       290                 295                 300
   Arg Leu Trp Gln Gln Lys Asn Glu Gln Ile Phe Ser Asn Val Thr His
   305                 310                 315                 320
   Cys Ala Met Ala Phe Arg Leu Leu Arg Met Ser Tyr Tyr Asp Val Ser
                   325                 330                 335
   Ser Asp Glu Leu Ala Glu Phe Val Asp Glu Glu His Phe Phe Ala Ile
   340 345 350
   Ser Gly Lys Tyr Thr Ser His Val Glu Ile Leu Glu Leu His Lys Ala
   355 360 365
   Ser Gln Leu Ala Ile Asp His Glu Lys Asp Asp Ile Leu Asp Lys Ile
   370 375 380
   Asn Asn Trp Thr Arg Thr Phe Met Glu Gln Lys Leu Leu Asn Asn Gly
   385 390 395 400
   Phe Ile Asp Arg Met Ser Lys Lys Glu Val Glu Leu Ala Leu Arg Lys
   405 410 415
   Phe Tyr Thr Ile Ser Asp Leu Ala Glu Asn Arg Arg Cys Ile Lys Ser
   420 425 430
   Tyr Glu Glu Asn Asn Phe Lys Ile Leu Lys Ala Ala Tyr Arg Ser Pro
   435 440 445
   Asn Ile Tyr Asn Lys Asp Leu Phe Ile Phe Ser Ile Arg Asn Phe Glu
   450 455 460
   Leu Cys Gln Ala Gln His Gln Glu Glu Leu Gln Gln Phe Lys Arg Trp
   465 470 475 480
   Phe Glu Asp Tyr Arg Leu Asp Gln Leu Gly Ile Ala Glu Arg Tyr Ile
   485 490 495
   His Asp Thr Tyr Leu Cys Ala Val Ile Val Val Pro Glu Pro Glu Leu
   500 505 510
   Ser Asp Ala Arg Leu Leu Tyr Ala Lys Tyr Val Leu Leu Leu Thr Ile
   515 520 525
   Val Asp Asp Gln Phe Asp Ser Phe Ala Ser Thr Asp Glu Cys Leu Asn
   530 535 540
   Ile Ile Glu Leu Val Glu Arg Trp Asp Asp Tyr Ala Ser Val Gly Tyr
   545 550 555 560
   Lys Ser Glu Lys Val Lys Val Phe Phe Ser Thr Leu Tyr Lys Ser Ile
   565 570 575
   Glu Glu Leu Val Thr Ile Ala Glu Ile Lys Gln Gly Arg Ser Val Lys
   580 585 590
   Asn His Leu Leu Asn Leu Trp Leu Glu Leu Val Lys Leu Met Leu Met
   595 600 605
   Glu Arg Val Glu Trp Phe Ser Gly Lys Thr Ile Pro Ser Ile Glu Glu
   610 615 620
   Tyr Leu Tyr Val Thr Ser Ile Thr Phe Gly Ala Arg Leu Ile Pro Leu
   625 630 635 640
   Thr Thr Gln Tyr Phe Leu Gly Ile Lys Ile Ser Glu Asp Ile Leu Glu
   645 650 655
   Ser Asp Glu Ile Tyr Gly Leu Cys Asn Cys Thr Gly Arg Val Leu Arg
   660 665 670
   Ile Leu Asn Asp Leu Gln Asp Ser Lys Lys Glu Gln Lys Glu Asp Ser
   675 680 685
   Val Thr Ile Val Thr Leu Leu Met Lys Ser Met Ser Glu Glu Glu Ala
   690 695 700
   Ile Met Lys Ile Lys Glu Ile Leu Glu Met Asn Arg Arg Glu Leu Leu
   705 710 715 720
   Lys Met Val Leu Val Gln Lys Lys Gly Ser Gln Leu Pro Gln Ile Cys
   725 730 735
   Lys Asp Ile Phe Trp Arg Thr Ser Asn Trp Ala Asp Phe Ile Tyr Leu
   740 745 750
   Gln Thr Asp Gly Tyr Arg Ile Ala Glu Glu Met Lys Asn His Ile Asp
   755 760 765
   Glu Val Phe Tyr Lys Pro Leu Asn His
   770 775
<210> 5
   <211> 31
   <212> DNA
   <213> artificial sequence
<220>
   <223> amorce
<400> 5
   accatgggtt ctttggttct tcaatgttgg a 31
<210> 6
   <211> 34
   <212> DNA
   <213> artificial sequence
<220>
   <223> amorce
<400> 6
   actcgagata tgtgtgtcca ccaaaacgtc tatg 34
<210> 7
   <211> 31
   <212> DNA
   <213> artificial sequence
<220>
   <223> amorce
<400> 7
   tccatggtag ttggctatag aagcacaatc a 31
<210> 8
   <211> 34
   <212> DNA
   <213> artificial sequence
<220>
   <223> amorce
<400> 8
   tctcgagcat aaattcaaat tgagggatta atga 34
<210> 9
   <211> 31
   <212> DNA
   <213> artificial sequence
<220>
   <223> amorce
<400> 9
   accatgggtt ctttggttct tcaatgttgg a 31
<210> 10
   <211> 34
   <212> DNA
   <213> artificial sequence
<220>
   <223> amorce
<400> 10
   actcgagata tgtgtgtcca ccaaaacgtc tatg 34

## Description

### Domaine de l'Invention

La présente invention se rapporte à deux gènes responsables de la synthèse d'un mélange de sesquiterpènes et leur utilisation pour la préparation de ces composés dans des organismes vivants tels que les bactéries, les levures, les cellules animales et les plantes.

### Introduction

Les terpènes sont des molécules présentes dans tous les organismes vivants tels que les bactéries, les champignons, les animaux, et les plantes. Ils forment la plus grande classe de molécules naturelles du monde vivant. Chez les plantes, ces molécules sont présentes dans le métabolisme primaire en tant qu'hormones (les gibbérellines, les cytokinines, les brassinostéroïdes et l'acide abscissique) ou en tant que composés impliqués dans la photosynthèse (caroténoïdes, chlorophylles, plastoquinones et phytol) ainsi que dans les processus de prénylation des protéines, et dans la structure des membranes (stérols). Cependant, les terpènoïdes issus du métabolisme secondaire représentent l'essentiel de la diversité structurale de cette classe de molécules. Les rôles des terpènoïdes dits secondaires se rangent essentiellement dans le domaine des interactions avec l'environnement comme par exemple l'attraction d'insectes bénéfiques pour la plante (pollinisation et prédateurs d'insectes phytophages), la défense contre les pathogènes et les insectes et la protection contre le stress photo-oxydatif (Tholl, 2006).

Les terpènes sont constitués de multiples d'unités isoprényles à 5 carbones. Le nombre d'unités isoprényles permet de les classer en monoterpènes (composés à dix atomes de carbone ou C10), sesquiterpènes (C15), diterpènes (C20), sesterterpènes (C25), triterpènes (C30), tetraterpènes (C40), et ainsi de suite. Les précurseurs universels de tous les terpènes sont l'isopentenyl diphosphate (IPP) et le diméthylallyl diphosphate (DMAPP). Deux voies distinctes conduisent à la formation de l'IPP et du DMAPP. L'une, la voie du mévalonate (MEV) est localisée dans le cytoplasme des cellules eucaryotes alors que l'autre, la voie du méthyl-erythritol (MEP) n'existe que chez certaines bactéries et les plantes où elle est localisée dans le chloroplaste (Rodriguez-Concepcion et Boronat, 2002). Toutes les étapes et tous les gènes codant les enzymes des étapes de ces deux voies sont connus chez un certain nombre d'organismes.

La première étape de la biosynthèse des terpènes à partir des précurseurs communs IPP et DMAPP est réalisée par des prényl diphosphate synthases (ou prényltransférases) qui vont par condensation d'un précurseur homoallylique, l'IPP, et d'un précurseur allylique (le DMAPP, le géranyl diphosphate, le farnésyldiphosphate ou encore le géranylgéranyldiphosphate), générer des chaînes de prényldiphosphates de longueur variable. Il existe deux grandes classes principales de prényldiphosphate synthases : les *cis*-prényltransférases ou *Z*-prényltransférases et les *trans*-prényltransférases ou *E-*prényltransférases. Les *cis*-prényltransférases conduisent à la formation d'une double liaison en configuration *cis,* alors que les *trans*-prényltransférases conduisent à la formation d'une double liaison en configuration *trans* (Koyama 1999). Ainsi, l'addition séquentielle de 2 unités d'IPP sur un DMAPP par une *trans*-farnésyldiphosphate synthase, ou *E*-FPS produit du *E,E*-farnésyldiphosphate (*E,E*-FPP, C15). La réaction d'élongation commence par la formation d'un cation allylique après l'élimination de l'ion diphosphate pour former un prényl allylique. Ensuite, l'addition d'un IPP se produit avec une élimination stéréospécifique d'un proton en position 2 pour former une nouvelle liaison C-C et une nouvelle double liaison dans le produit. La répétition de cette condensation stéréospécifique de l'IPP avec un prényl diphosphate allylique, conduit à la synthèse d'un prényl diphosphate de longueur de chaîne et de stéréochimie données spécifiques de chaque enzyme. En particulier, la longueur de la chaîne du produit final peut varier considérablement allant d'un C10 (géranyl diphosphate) jusqu'aux polyprenyldiphosphates précurseurs du latex naturel composés de plusieurs milliers d'atomes de carbone.

Comme mentionné plus haut, les prényltransférases peuvent être divisées en deux familles génétiques différentes selon la stéréochimie (*E* ou *Z*) de la double liaison formée après chaque cycle d'élongation (Poulter, 2006 ; Liang *et al.,* 2002).

Les *E-*prényltransférases caractérisées à ce jour sont responsables de la synthèse de prényl phosphates à courte chaîne (C10 à C50) en configuration *E*. On peut citer par exemple la géranyl diphosphate synthase (GPS), la famesyl diphosphate synthase (FPS), la géranyl geranyl diphosphate synthase (GGPS), l'octaprenyl diphosphate synthase (OPS), la solanesyl diphosphate synthase (SPS) et la decaprenyl diphosphate synthase (DPS) qui sont respectivement responsables de la synthèse du *(E)*-GPP (C10), (*E,E*)-FPP (C15), (*E,E,E*)-GGPP (C20), *(E,E,E,E,E,E,E)-*OPP (C40), *(E,E,E,E,E,E,E,E)-*SPP (C45), et du (*E*, *E,E,E,E,E,E,E,E)*-DPP (C50). Il faut noter que le (*E,E*)-FPP est en général le substrat des prényltransférases responsables de la synthèse des chaînes à plus de 20 carbones. Le premier gène codant pour une (*E*)-prényl transférase caractérisé est celui de la FPS de rat (Clarke *et al.,* 1987). L'alignement des séquences codant pour des *E*-prényltransférases a permis de mettre en évidence deux motifs conservés de type DDXXD. La structure tridimensionnelle d'une *E*-prényltransférase, déterminée aux rayons X pour la première fois sur une FPS (Tarshis *et al.,* 1994), et les études de mutagenèse dirigée ont montré que les deux motifs DDXXD étaient impliqués dans la liaison avec les substrats et dans l'activité catalytique en résultant. Finalement, il a été démontré qu'un petit nombre d'acides aminés spécifiques, localisés en amont du premier motif DDXXD, déterminaient la longueur de l'élongation de la chaîne d'isoprényles synthétisée (Wang & Ohnuma, 1999).

Le premier gène codant pour une *Z*-prényltransferase a été cloné chez *Micrococus luteus* (Shimizu *et al.,* 1998). Il code pour une undecaprényltransférase (UPS) qui utilise le *E*-FPP comme substrat pour former un prényl diphosphate à 55 carbones qui possède une stéréochimie de type *Z*,*E*. Cette molécule intervient dans la biosynthèse des peptidoglycanes de la paroi de certaines bactéries. Une séquence homologue a été caractérisée chez *Arabidopsis* comme étant responsable de la synthèse d'isoprényl diphosphate en conformation *Z*,*E* de 100 à 130 carbones (Oh *et al.,* 2000). L'analyse des séquences en acides aminés de ces enzymes ne montre aucune homologie avec les séquences des *E*-prényltransferases (Koyama, 1999). En particulier, les *Z-*prényltransferases ne possèdent pas de motifs riches en acide aspartique (DDXXD). Sept régions conservées qui interviennent toutes plus ou moins dans la fixation du substrat et la catalyse de celui-ci ont été identifiées. Toutes les *Z*-prényltransferases identifiées à ce jour synthétisent des prényls diphosphates ayant une longueur de chaîne supérieure ou égale à 55 carbones à l'exception d'une *Z,E*-FPS de *Mycobacterium tuberculosis* qui utilise le *E-*GPP comme substrat pour former du *Z,E*-FPP (Schulbach *et al.,* 2000). Une étude récente de mutagenèse dirigée sur l'UPS de *M. luteus* a permis d'identifier plusieurs acides aminés jouant un rôle clé dans le nombre de cycles d'élongation de la chaîne d'isoprényles synthétisée (Kharel *et al.,* 2006).

Les isoprényles diphosphates sont les substrats d'une classe d'enzyme, les terpènes synthases, qui permettent la formation du squelette de base des terpènes cycliques ou acycliques en C10 (monoterpène), C15 (sesquiterpène), C20 (diterpène), et C30 (triterpène) (Cane 1999; McMillan and Beale, 1999; Wise and Croteau, 1999). La diversité de réaction de ces enzymes est à l'origine de la diversité des squelettes terpéniques cycliques que l'on retrouve dans la nature. Quatre stéréoisomères du farnésyl diphosphate sont théoriquement possibles en fonction de la configuration stéréochimique des doubles liaisons en position 2 ou 6 de la molécule (*E*,*E*, *Z,E, E,Z ou Z,Z).* Cependant, à ce jour, toutes les sesquiterpènes synthases pour lesquelles les gènes ont été caractérisés utilisent comme substrat le *E,E*-FPP. C'est le cas par exemple de la 5-épi-aristolochene synthase de tabac (Facchini & Chappell, 1992), de la (*E*)-alpha-bisabolene synthase d'*Abies grandis* (Bohlmann *et al.,* 1998), de la germacrene A synthase de chicorée (Bouwmeester *et al.,* 2002), de l'amorpha-4,11-diene synthase de l'armoise annuelle (Wallaart *et al.,* 2001), de la germacrene C synthase de tomate (Colby *et al.,* 1998), de la caryophyllene synthase de l'armoise annuelle (Cai *et al.,* 2002), et de la valencène synthase d'orange (Sharon-Asa *et al.,* 2003). Une étude récente a cependant montré qu'une sesquiterpène synthase de maïs (tps4) responsable de la synthèse d'un mélange de 14 sesquiterpènes acceptait le *E*,*E*-FPP et le *Z,E*-FPP (Kollner *et al.,* 2006), montrant ainsi que les sesquiterpènes synthases pouvaient utiliser d'autres isomères que le *E,E*-FPP comme substrat. Néanmoins, aucune sesquiterpène synthase clonée à ce jour n'utilise de *Z,Z*-FPP.

Les trichomes glandulaires de type VI de *Solanum habrochaites* (auparavant dénommée *Lycopersicum hirsutum*) excrètent de grandes quantités de sesquiterpènes oléfines appartenant à deux classes distinctes. La classe I contient notamment la germacrène B et la classe II contient entre autres l'alpha-santalène et l'alpha-bergamotène. L'analyse de la ségrégation entre un génotype de tomate cultivée (*Lycopersicon esculentum = Solanum lycopersicum*) et un génotype de tomate sauvage (*Lycopersicon hirsutum = Solanum habrochaites*) a permis de démontrer que la biosynthèse de ces deux classes différentes de sesquiterpènes était contrôlée par deux loci différents (SstlA et Sst2) localisés sur deux chromosomes différents (van der Hoeven *et al.* 2000). Le locus SstlA est localisé sur le chromosome 6, et les gènes de ce locus sont responsables de l'accumulation des sesquiterpènes de classe I. L'allèle Sst2 de *S*. *habrochaites* localisé sur le chromosome 8 contrôle l'accumulation de sesquiterpènes de classe II comme l'alpha-santalène mais aussi le cis-alpha-bergamotène, le trans-alpha-bergamotène, l'epi-beta-santalène et le beta-bergamotène et d'autres composés mineurs non identifiés. Tandis que les séquences codantes correspondant aux gènes localisés sur le locus Sstl-A ont été identifiées (van der Hoeven *et al.* 2000), celles localisées sur le locus Sst2 ne l'ont pas été.

WO2004/111183 et ADW26108 de la base de données EMBL décrivent une séquence nucléotidique (SEQ ID No 70) à laquelle aucune fonction n'a été associée. AW616634 de la base de données EMBL décrit une séquence à laquelle aucune fonction n'a été associée. Enfin, Adams et al (PHYTOCHEMISTRY, vol. 12, no. 9, 1973, pages 2167-2172) décrit l'isolement d'une fraction protéique possédant une activité Z,Z-FPP synthase.

### Résumé de l'Invention

La présente invention se rapporte à deux gènes responsables de la synthèse d'un mélange de sesquiterpènes composé principalement d'alpha-santalène, d'épi-beta-santalène, de cis-alpha-bergamotène, de trans-alpha-bergamotène, et de endo-beta-bergamotène et de leur précurseur le *Z,Z*-farnesyl diphosphate (*Z,Z-*FPP) et leur utilisation pour la préparation de ces composés dans des organismes vivants tels que les bactéries, les levures, les cellules animales et les plantes.

Plus précisément, le présent document décrit l'identification et la caractérisation de deux gènes de *S*. *habrochaites* responsables de la synthèse des sesquiterpènes de la classe II (alpha-santalène, épi-beta-santalène, cis-alpha-bergamotène, trans-alpha-bergamotène et endo-beta-bergamotène entre autres) de *S*. *habrochaites* que nous avons renommés sesquiterpènes de type SB (pour Santalène et Bergamotène) dans la suite du texte. Le premier gène code pour une *Z*,*Z*-farnesyl diphosphate synthase et le second pour une sesquiterpène synthase à produits multiples qui utilise le *Z,Z*-farnesyl diphosphate comme substrat. La production des protéines recombinantes correspondantes nous a permis de démontrer que le profil sesquiterpènique obtenu *in vitro* est identique à celui du contrôlé par le locus Sst2 de tomate.

L'invention est relative aux activités enzymatiques de la biosynthèse du *Z,Z*-FPP à partir d'IPP et de DMAP d'une part, et d'un mélange de sesquiterpènes composé principalement d'alpha-santalène, d'épi-beta-santalène, de cis-alpha-bergamotène, de trans-alpha-bergamotène et de endo-beta-bergamotène à partir de *Z,Z*-FPP, les séquences peptidiques responsables de ces activités et les séquences d'acide nucléique codant ces séquences peptidiques. L'invention se rapporte également aux méthodes utilisant ces activités enzymatiques pour la production de ces composés ou de dérivés de ceux-ci.

La présente invention concerne donc une méthode de production d'un mélange de sesquiterpènes, principalement composés d'alpha-santalène, d'épi-beta-santalène, de cis-alpha-bergamotène, de trans-alpha-bergamotène et de endo-beta-bergamotène, à partir de *Z,Z*-FPP dans une cellule ayant une source d'IPP et de DMAP, comprenant :
a) l'introduction dans ladite cellule d'une construction portant une cassette d'expression comprenant un premier gène codant pour une *Z,Z-*FPS présentant au moins 80 % d'identité avec la SEQ ID No 2 et d'une construction portant une cassette d'expression comprenant un deuxième gène codant pour une sesquiterpène synthase dénommée SBS présentant au moins 80 % d'identité avec la SEQ ID No 4;
b) la culture de la cellule transformée dans des conditions appropriées pour l'expression desdits premier et deuxième gènes ; et,
c) facultativement, la récolte du *Z,Z-*FPP ou des produits sesquiterpèniques de l'enzyme SBS ou de dérivés de ceux-ci contenus dans ladite cellule et/ou dans le milieu de culture.

La présente invention concerne une protéine isolée ou recombinante ayant une activité *Z,Z-*FPS et une séquence présentant au moins 80 % d'identité avec la SEQ ID No 2. La description décrit aussi un acide nucléique comprenant une séquence nucléotidique codant pour une telle protéine, ou une séquence capable de s'hybrider à celui-ci dans des conditions stringentes.

La présente invention concerne également une protéine isolée ou recombinante ayant une activité de type SB synthase et ayant une séquence présentant au moins 80 % d'identité avec la SEQ ID No 4. La description décrit aussi un acide nucléique comprenant une séquence nucléotidique codant pour une telle protéine, ou une séquence capable de s'hybrider à celui-ci dans des conditions stringentes.

La présente invention concerne une cassette d'expression comprenant un acide nucléique hétérologue codant pour une protéine selon la présente invention, un vecteur comprenant une cassette d'expression ou un acide nucléique hétérologue codant pour une protéine selon la présente invention, une cellule hôte comprenant un vecteur, une cassette d'expression ou un acide nucléique comprenant une séquence hétérologue codant pour une protéine selon la présente invention, et un organisme transgénique non-humain comprenant une cellule, un vecteur, une cassette d'expression ou un acide nucléique comprenant une séquence hétérologue codant pour une protéine selon la présente invention.

La présente invention concerne en outre une méthode de production du *Z,Z-*farnesyl diphosphate (*Z,Z*-FPP), ou de dérivés de ceux-ci à partir d'IPP et de DMAP dans une cellule ayant une source d'IPP et de DMAP, comprenant :
a) l'introduction dans ladite cellule d'une construction portant une cassette d'expression avec une séquence nucléique codant une *Z,Z*-FPS selon la présente invention ;
b) la culture de la cellule transformée dans des conditions appropriées pour l'expression du gène ; et
c) facultativement, la récolte du ou de dérivés de celui-ci contenus dans ladite cellule et/ou dans le milieu de culture.

La présente invention concerne l'utilisation d'une protéine, d'un acide nucléique, d'une cellule hôte ou d'un organisme transgénique selon la présente invention pour la préparation de *Z,Z-*FPP ou des sesquiterpènes de type SB, tels que l'alpha-santalène, l'épi-beta-santalène, le cis-alpha-bergamotène, le trans-alpha-bergamotène et l'endo-beta-bergamotène, ou de dérivés de ceux-ci comme l'alpha-santalol, l'épi-beta-santalol, le cis-alpha-bergamotol, le trans-alpha-bergamotol et le beta-bergamotol.

La présente invention concerne une cellule ou un organisme transgénique non-humain, caractérisé en ce que la voie de synthèse des sesquiterpènes de classe II est bloquée par inactivation soit du gène codant pour une *Z,Z-*FPS selon la présente invention, soit du gène codant pour une SB synthase selon la présente invention, soit encore des deux gènes.

La présente invention concerne en outre l'utilisation d'un acide nucléique pour identifier des marqueurs moléculaires permettant d'introduire les séquences génomiques correspondantes dans d'autres espèces ou variétés de tomate cultivées (*Solanum lycopersicum).* Au titre de la présente invention, les séquences nucléotidiques SEQ ID N°1 et N°2 ou des variants de celles-ci peuvent également être utilisées comme marqueurs moléculaires pour l'introgression de ces séquences dans d'autres espèces ou variétés de tomates cultivées.

Ainsi, la présente invention concerne des marqueurs moléculaires comprenant tout ou partie d'un acide nucléique présentant au moins 80 % d'identité avec la SEQ ID No 1 ou SEQ ID No 3 pour identifier un polymorphisme génomique entre *S. habrochaites* et des espèces de type *Solanum* sexuellement compatible avec *S*. *habrochaites* afin d'introduire les gènes correspondant dans ces espèces. Elle concerne également une méthode consistant à introduire les gènes *zFPS* et *SBS* dans une espèce non sexuellement compatible avec *Solanum habrochaites* par fusion de protoplastes.

La description décrit aussi une méthode de production du *Z,Z-*farnesol par déphosphorylation du Z,Z-FPP par une phosphatase.

### Légende des Figures

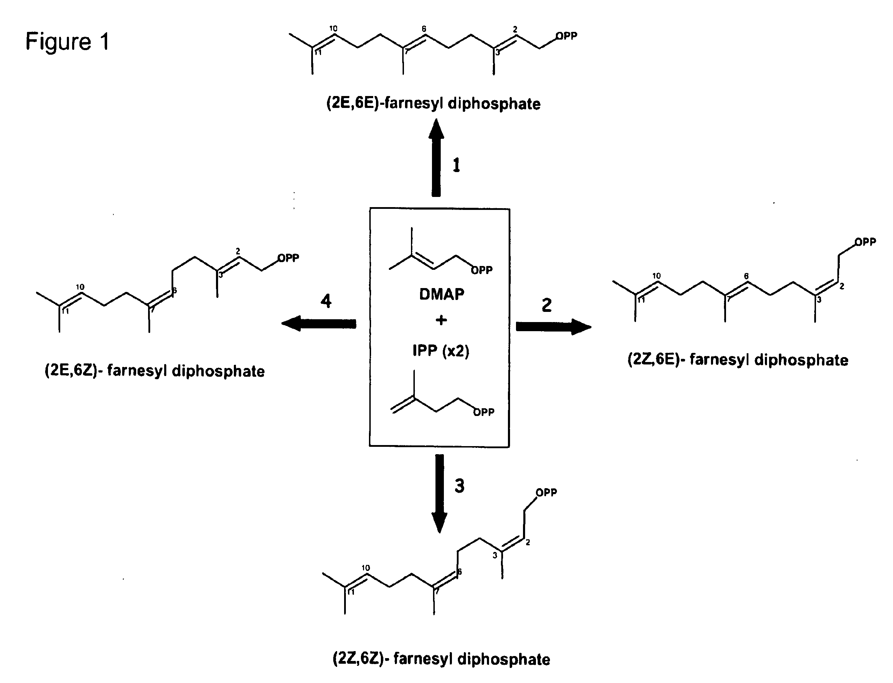

**Figure 1** **:** Les stéréosiomères possibles du FPP. Tous les stéréoisomères du FPP sont biosynthétisés à partir d'IPP et de DMAPP. A ce jour seules étaient connues la *E,E-*famesyl diphosphate synthase (1) caractérisée dans de nombreux organismes, et la *Z,E-*famesyl diphosphate synthase (2) de *Mycobacterium tuberculosis* (Schulbach *et al.,* 2000). (3) : *Z,Z*-farnesyl diphosphate synthase de tomate selon la présente invention ; (4) : *E,Z-*farnesyl diphosphate synthase : aucune enzyme ayant cette activité n'a été décrite à ce jour.

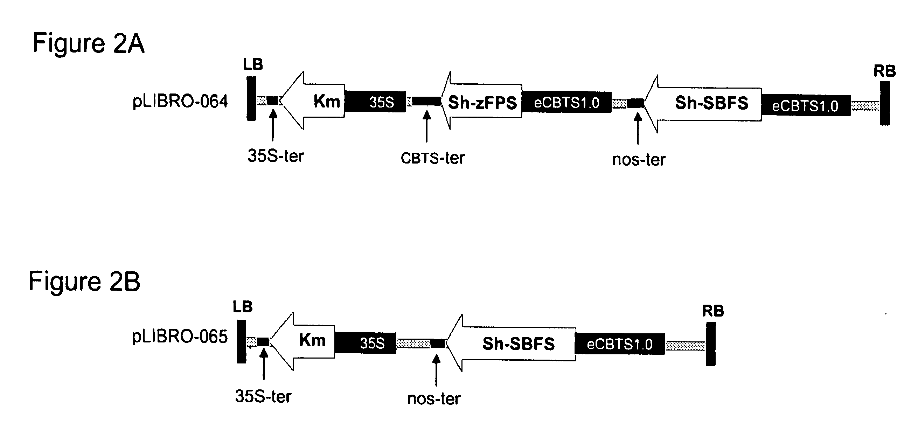

**Figure 2** **:** Représentation schématique des ADN-T portant les transgènes *Sh-zFPS* et *Sh-SBS. Km* : gène de résistance à la kanamycine ; 35S : promoteur du gène 35S du CaMV. CBTS-ter: terminateur du gène CBTS; eCBTS1.0 : promoteur de 1kb du gène CBTS fusionné avec l'amplificateur du promoteur 35S du CaMV. *Sh-zFPS :* phase codante du gène codant pour la zFPS de tomate (*S. habrochaites LA1777*) *; Sh-SBS* : phase codante du gène codant pour la SB synthase de tomate (*S. habrochaites LA1777*); LB : Bordure gauche du T-DNA; RB : Bordure droite du T-DNA; 35S-ter : Terminateur du gène 35S du CaMV; nos-ter : Terminateur du gène de la nopaline synthase d*'Agrobacterium tumefasciens.* **Figure 2** : fragment ADN-T du vecteur binaire pLIBRO64 ; **Figure 2B** **:** fragment ADN-T du vecteur binaire pLIBRO65.

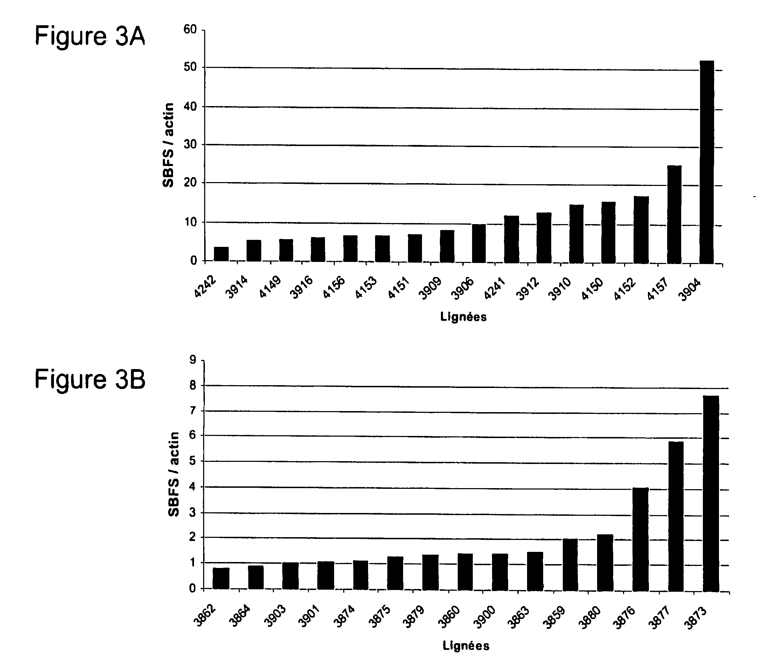

**Figure 3** **:** Analyse de l'expression du transgène *Sh-SBS* dans les feuilles de tabac transgéniques. L'expression a été mesurée par PCR quantitative en temps réel en utilisant des sondes fluorescente (®TAQ-MAN, ABI). L'une des sondes est spécifique du transgène *Sh-SBS* tandis que l'autre est spécifique d'un gène d'actine de tabac (gène contrôle). Les valeurs de l'axe des ordonnées représentent le rapport des puissances 2 des valeurs obtenues avec la sonde *Sh-SBS* sur celles obtenues avec la sonde actine(2^{SBS}/2^{actine}). Ce rapport exprime le rapport d'expression du transgène *Sh-SBS* sur celui de l'actine. Les valeurs de l'axe des abscisses indiquent le numéro des lignées transgéniques. **Figure 3A**) Lignées de tabac transgéniques ayant intégré les transgènes *Sh-zFPS* et *Sh-SBS* (pLIBRO-064) **Figure 3B**) Lignées de tabac transgéniques ayant intégré uniquement le transgène *Sh-SBS* (pLIBRO-065).

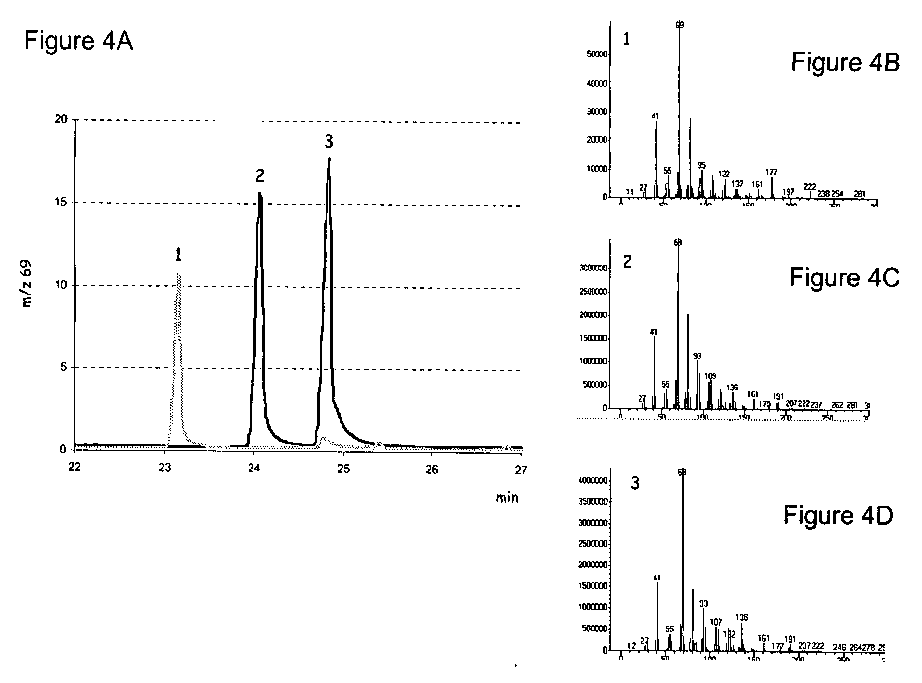

**Figure 4** **:** Profil GC/MS du (*Z,Z*)-farnesol superposé aux standard de farnesols (mélange d'isomères). **Figure 4A** **:** Le chromatogramme gris (m/z: 69) correspond au produit obtenu *in vitro* avec la protéine recombinante *Sh-zFPS-*6His*. Sh*-FPS-6His a été incubée avec du DMAPP et de l'IPP. Le produit isoprényl-diphosphate a été déphosphorylé en un alcool et purifié par chromatographie en phase liquide. Le pic n° 1 correspond au *Z,Z*-farnesol. 1. Le chromatogramme noir (m/z: 69) correspond au standard farnesol constitué d'un mélange d'isomères (*Z,E*)-, (*E,Z*)-, et (*E,E*)-farnesol (Fluka). Le pic n°2 correspond à un mélange de (Z,E)- et (E,Z)- farnesol et le pic n°3 au (E,E)-farnesol. **Figure 4B** **:** Spectre de masse du (*Z,Z*)-farnesol correspondant au pic n°1. **Figure 4C** **:** Spectre de masse du (*Z,E*)- et du (*E,Z*)-farnesol (mélange) correspondant au pic n°2. **Figure 4D** **:** Spectre de masse du (*E,E*)-farnesol. correspondant au pic n°3.

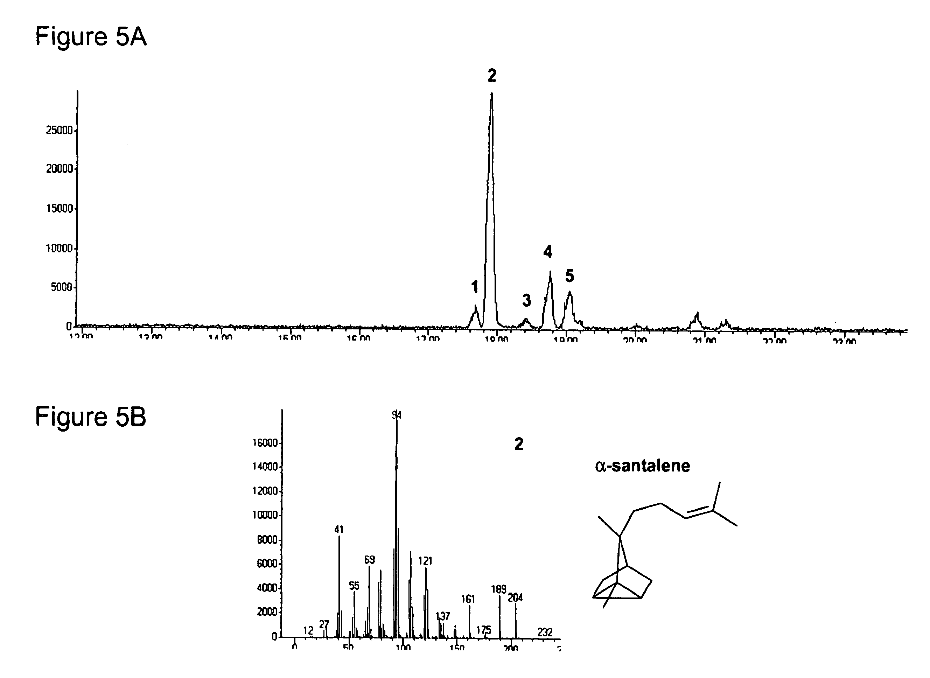

**Figure 5** **:** Profil GC/MS des tests d'activités *in vitro* réalisés avec les protéines recombinantes *Sh-zFPS*-6His et *Sh-SBS*-6His : Les deux enzymes recombinantes *Sh-zFPS-*6His et *Sh-SBS*-6His ont été incubées ensembles avec IPP et DMAPP. Le mélange réactionnel a été extrait au pentane et analysé par GC-MS. **Figure 5A** **:** Chromatogramme du produit obtenu *in vitro* de la *Sh*-zFPS et *Sh*-SBS Le pic **2** est le produit majoritaire et correspond à l'alpha-santalène.. **1,** *cis*-alpha-bergamotène ; **2,** alpha-santalène; **3,** *trans-*alpha-bergamotène; **4,** épi-beta-santalène; **5,** endo-beta-bergamotène. **Figure 5B** **:** Spectre de masse correspondant au pic n° 2 (à gauche) et structure de l'alpha-santalène (à droite).

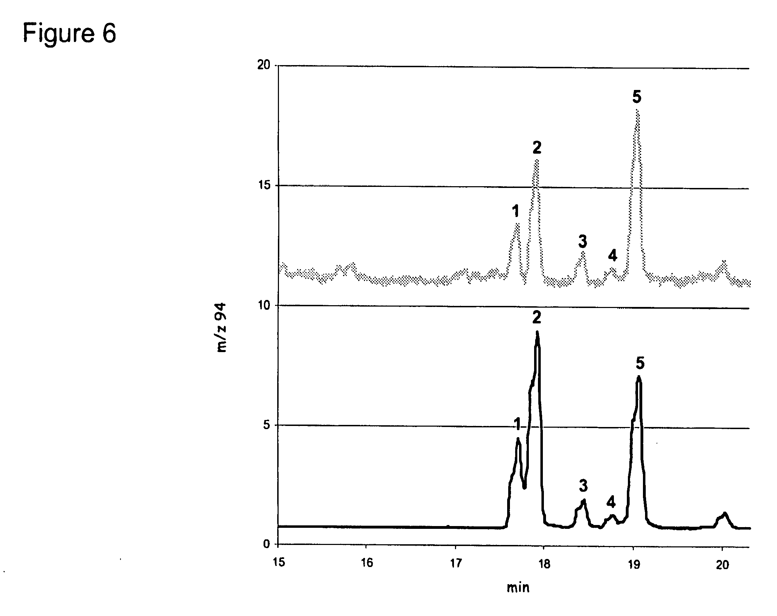

**Figure 6** **:** Superposition des profils GC entre les produits obtenus *in vitro* avec les enzymes recombinantes *Sh-zFPS-*6His et *Sh-SBS-*6His*,* et l'exsudat de la lignée recombinante isogénique de tomate TA517. Les tracés correspondent à l'ion extrait m/z 94. En gris: oléfines produites *in vitro* par *Sh-zFPS* et *Sh-*KS*.* En noir: exsudat de la lignée isogénique TA517. **1,** *cis*-alpha-bergamotène **2,** alpha-santalène; **3,** *trans*-alpha-bergamotène ; **4,** épi-beta-santalène; **5,** endo-beta-bergamotène.

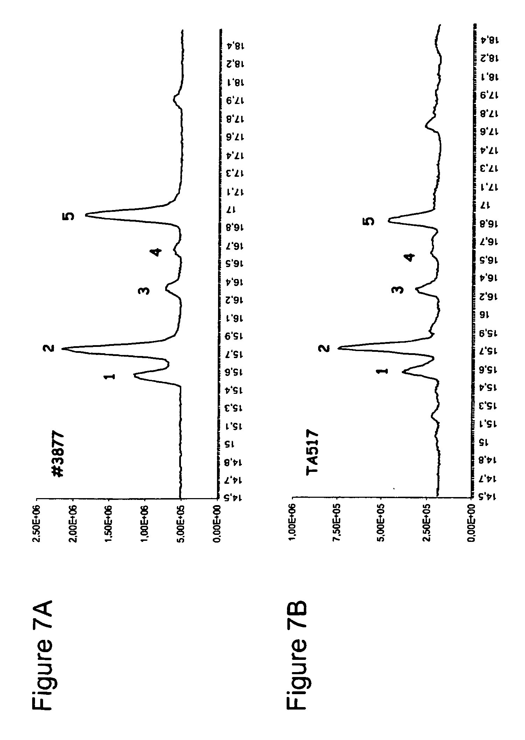

**Figure 7** **:** Profil GC/MS des molécules volatiles émises par une plante transgénique (#3877) portant les transgènes *Sh-zFPS* et *Sh-SBS.* La plante transgénique #3877 a été cultivée pendant 24 heures en atmosphère contrôlée dans une enceinte de culture. Les molécules volatiles émises par les plantes ont été piégées sur une matrice Super® Q (Alltech) et analysées par GC/MS. Le chromatogramme **(7A)** montre plusieurs pics ayant des signatures m/z caractéristiques des sesquiterpènes de type SB. Les pics ont été identifiés par comparaison de leur temps de rétention et de leur spectre de masse avec ceux de la lignée TA517 **(7B). 1,** cis-alpha-bergamotène **2,** alpha-santalène; **3,** trans-alpha-bergamotène ; **4,** épi-beta-santalène; **5,** endo-beta-bergamotène.

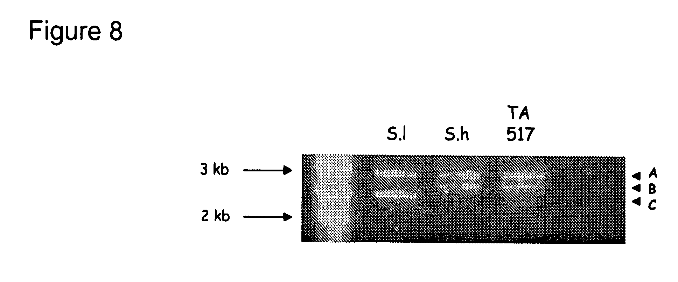

**Figure 8** **:** Polymorphisme du gène *zFPS* entre *S*. *lycopersicum* (*Sl*) et *S*. *habrochaites* (*Sh*): Le gène complet *zFPS* a été amplifié par PCR à partir d'ADN génomique de *S*. *lycopersicum, S. habrochaites* et TA517. La séparation des produits PCR sur gel d'agarose à 0,8% permet d'identifier 3 bandes A, B, C d'une taille d'environ 3000, 2500 et 2000 nucléotides respectivement. Le produit PCR correspondant à la bande B est spécifique du génome *S.h* et correspond au gène *zFPS* présenté dans ce document. kb : kilobases

### Abréviations et Définitions

ARNm : acide ribonucléique messager
ADN : acide désoxyribonucléique
ADNc : ADN complémentaire produit par transcription inverse à partir d'ARNm.
CaMV : virus de la mosaïque du chou-fleur
DMAPP : diméthylallyl diphosphate
CBT-ol : cembratrien-ol
CBTS : cembratrien-ol synthase
GPP : Geranyl diphosphate
FPP : Farnesyl diphosphate
GC : chromatographie gazeuse
GGPP : géranyl géranyl diphosphate
ihpRNAi : intron hairpin ARN interférent
IPP : isopentenyl diphosphate
MS : spectrométrie de masse
PCR : "polymerase chain reaction"
RACE : "rapid amplification of cDNA ends"
A, R, N, D, C, Q, E, G, H, I, L, K, M, F, P, S, T, W, Y, V : Code standard des acides aminés selon la nomenclature universelle
www3.ncbi.nlm.nih.gov/Taxonomy/Utils/wprintgc.cgi?mode=t
A,C,G,T, B,D,H,K,M,N,R,S,V,W,Y : Code standard des bases selon la nomenclature universelle www3.ncbi.nlm.nih.gov/Taxonomy/Utils/wprintgc.cgi?mode=t RFLP : « Restriction fragment length polymorphism »

Par « *Z,Z-*farnesyl diphosphate synthase » ou zFPS est entendue une enzyme capable de produire du *Z,Z*-farnesyl diphosphate (*(2Z,6Z)*-farnesyldiphosphate ou *cis,cis-FPP*)*,* à partir d'isopentenyl diphosphate (IPP) et de dimethyl allyl diphosphate (DMAP). Par « activité *Z,Z*-FPS » est entendue la production de *Z,Z-* Famesyl diphosphate à partir de IPP et DMAPP. L'activité *Z,Z*-FPS peut être mesurée en mesurant l'apparition du *Z,Z-*farnesyl diphosphate.

Par « SB synthase » ou SBS est entendue une enzyme capable de produire un mélange de sesquiterpènes principalement composé d'alpha-santalène, d'épi-beta-santalène, de cis-alpha-bergamotène, de trans-alpha-bergamotène et de endo-beta-bergamotène à partir de *Z,Z*-FPP. Par « activité de type SB » est entendue la production de sesquiterpènes de type SB à partir de *Z,Z-* Farnesyl diphosphate. L'activité de type SB peut être mesurée en mesurant l'apparition d'un ou plusieurs sesquiterpènes de type SB. Un exemple de mesure d'activité de type SB est décrit dans les exemples.

Par « sesquiterpène de type SB » est entendue un mélange de sesquiterpènes principalement composé d'alpha-santalène, d'épi-beta-santalène, de cis-alpha-bergamotène, de trans-alpha-bergamotène et de endo-beta-bergamotène.

« Conditions stringentes d'hybridation » : Généralement, des conditions stringentes, pour un polynucléotide de taille et de séquence données, sont obtenues en opérant a une température inférieure d'environ 5°C a 10°C à la température de fusion (Tm) de l'hybride formé, dans le même mélange réactionnel, par ce polynucléotide et son complémentaire.

Des conditions stringentes d'hybridation pour un polynucléotide donné, peuvent être identifiées par l'homme du métier en fonction de la taille et de la composition en bases du polynucléotide concerné, ainsi que de la composition du mélange d'hybridation (notamment pH et force ionique). Des conditions de forte stringence incluent une étape de lavage avec du tampon 0,2 x SSC à 65°C.

Les conditions d'hybridations de stringences décrites ci-dessus peuvent être adaptées par l'homme du métier pour les polynucléotides de taille plus grande ou plus petite, selon les enseignements appropriés connus de l'homme du métier, notamment décrit dans Sambrook et al., 1989, Molecular cloning, A laboratory Manual, Cold Spring Harbor ; Maniatis et al., 1982, Molecular cloning, A laboratory Manual, Cold Spring Harbor Lab. CSH, N. Y. USA, ou l'une de ses récentes rééditions, et dans Ausubel et al., Eds., 1995, Current Protocols in Molecular Biology, Chapter 2 (Greene Publishing and Wiley-Interscience, N. Y.).

Par « hétérologue » est entendu que le gène a été introduit par génie génétique dans la cellule. Il peut y être présent sous forme épisomale ou chromosomique. L'origine du gène peut être différente de la cellule dans laquelle il est introduit. Cependant, le gène peut également provenir de la même espèce que la cellule dans laquelle il est introduit mais il est considéré comme hétérologue en raison de son environnement qui ne sera pas naturel. Par exemple, le gène est dit hétérologue car il est sous le contrôle d'un promoteur autre que son promoteur naturel, il est introduit dans un endroit différent de celui-ci où il est situé naturellement. La cellule hôte peut contenir une copie du gène endogène au préalable de l'introduction du gène hétérologue ou elle peut ne pas contenir de copie endogène.

Par « pourcentage d'identité » entre deux séquences d'acide nucléique ou d'acides aminés au sens de la présente invention, on entend désigner un pourcentage de nucléotides ou de résidus d'acides aminés identiques entre les deux séquences à comparer, obtenu après le meilleur alignement, ce pourcentage étant purement statistique et les différences entre les deux séquences étant réparties au hasard et sur toute leur longueur. Le meilleur alignement ou alignement optimal est l'alignement pour lequel le pourcentage d'identité entre les deux séquences à comparer, comme calculé ci- après, est le plus élevé. Les comparaisons de séquences entre deux séquences d'acide nucléique ou d'acides aminés sont traditionnellement réalisées en comparant ces séquences après les avoir alignées de manière optimale, ladite comparaison étant réalisée par segment ou par fenêtre de comparaison pour identifier et comparer les régions locales de similarité de séquence. L'alignement optimal des séquences pour la comparaison peut être réalisé, outre manuellement, au moyen de l'algorithme d'homologie locale de Smith et Waterman (1981) (Ad. App. Math. 2 : 482), au moyen de l'algorithme d'homologie locale de Neddleman et Wunsch (1970) (J. Mol. Biol. 48 : 443), au moyen de la méthode de recherche de similarité de Pearson et Lipman (1988) (Proc. Natl. Acad. Sci. USA 85 : 2444), au moyen de logiciels informatiques utilisant ces algorithmes (GAP, BESTFIT, FASTA et TFASTA dans le Wisconsin Genetics Software Package, Genetics Computer Group, 575 Science Dr., Madison, WI). Le pourcentage d'identité entre deux séquences d'acide nucléique ou d'acides aminés est déterminé en comparant ces deux séquences alignées de manière optimale par fenêtre de comparaison dans laquelle la région de la séquence d'acide nucléique ou d'acides aminés à comparer peut comprendre des additions ou des délétions par rapport à la séquence de référence pour un alignement optimal entre ces deux séquences. Le pourcentage d'identité est calculé en déterminant le nombre de positions identiques pour lesquelles le nucléotide ou le résidu d'acide aminé est identique entre les deux séquences, en divisant ce nombre de positions identiques par le nombre total de positions dans la fenêtre de comparaison et en multipliant le résultat obtenu par 100 pour obtenir le pourcentage d'identité entre ces deux séquences.

### Description Détaillée de l'Invention

La présente invention décrit donc pour la première fois les gènes codant pour les enzymes impliquées dans la voie de synthèse de plusieurs sesquiterpènes. En particulier, la présente invention se rapporte à la caractérisation d'une sesquiterpène synthase de tomate à produits multiples (SB synthase) qui permet la production d'un mélange de sesquiterpènes dénommés composés de type SB et principalement composé d'alpha-santalène, d'épi-beta-santalène, de cis-alpha-bergamotène, de trans-alpha-bergamotène et de endo-beta-bergamotène à partir de *Z,Z-*FPP. En outre, elle concerne également la caractérisation de la *Z,Z*-farnesyl diphosphate synthase (zFPS) de tomate qui permet la production de *Z,Z-*FPP à partir d'IPP et de DMAP. Ces enzymes peuvent être utilisées pour la production de sesquiterpènes de type SB ou de *Z,Z-*FPP, *in vitro* ou *in vivo* dans des organismes transgéniques ou des cellules ou micro-organismes recombinants. Les organismes transgéniques, cellules ou micro-organismes tels que les bactéries, les levures, les champignons, les cellules animales, d'insectes ou de plantes et les plantes ou animaux transgéniques sont considérés. Elles permettent également la production de composés dérivés du *Z,Z*-FPP comme le *Z,Z-*farnesol. Les procédés de la présente invention sont plus particulièrement applicables à la production de *Z,Z-*FPP dans des microorganismes (bactérie, levure) et de composés de type SB dans les plantes possédant des trichomes glanduleux de type sécréteurs. La production du mélange de sesquiterpènes de type SB ou de leurs dérivés peut également être diminuée ou supprimée dans des plantes, par exemple la tomate, par des techniques d'extinction de gènes ou par mutagenèse. En outre, les séquences identifiées dans la présente invention sont utiles pour l'identification et/ou le clonage de gènes codant pour des enzymes ayant les mêmes activités dans d'autres espèces d'organismes, en particulier de plantes. Enfin, des marqueurs moléculaires polymorphiques peuvent être identifiés à partir des acides nucléiques codant pour la zFPS et la SB synthase et permettront de suivre l'introduction des séquences génomiques fonctionnelles correspondantes dans d'autres espèces ou variétés de tomate cultivées (*Solanum lycopersicum*).

Le *Z,Z-*FPP est un substrat potentiel de sesquiterpènes synthases qui n'est pas disponible actuellement commercialement. Si la majorité des sesquiterpène synthases caractérisées à ce jour utilise le *E,E*-FPP, il est évident par ailleurs que l'absence de *Z,Z-*FPP disponible commercialement a très largement limité son utilisation à des fins expérimentales. La cadinène synthase de coton est le seul exemple qui décrit l'utilisation préférentielle du Z,Z-FPP par une sesquiterpène synthase (Heinstein *et al.,* 1970). Dans ce cas le *Z,Z-*FPP a été produit par synthèse chimique, et la *Z,Z*-FPP synthase décrite dans ce brevet fournit un moyen de produire cette molécule de manière aisée et peu coûteuse.

Par ailleurs, les sesquiterpènes de type SB contiennent l'alpha-santalène, lui-même précurseur direct de l'alpha-santalol. L'alpha-santalol est un des composants caractéristiques de l'huile essentielle de bois de santal. L'huile de bois de santal est extrèmement prisée en parfumerie et son coût a très fortement augmenté depuis 10 ans en raison d'une surexploitation du bois de santal, principalement en Inde. Cette surexploitation fait peser une menace sur la viabilité de la ressource naturelle de bois de Santal. L'alpha-santalol peut être obtenu à partir de l'alpha santalène par une simple hydroxylation.

La présente invention concerne donc une méthode de production de sesquiterpènes de type SB ou de dérivés de ceux-ci à partir de *Z,Z*-FPP dans une cellule ayant une source de DMAP et IPPP, comprenant :
a) l'introduction dans ladite cellule d'une construction portant une cassette d'expression comprenant un premier gène codant pour une zFPS selon la présente invention et d'une construction portant une cassette d'expression comprenant un deuxième gène codant pour une SB synthase selon la présente invention;
b) la culture de la cellule transformée dans des conditions appropriées pour l'expression desdits premier et deuxième gènes ; et,
c) facultativement, la récolte de sesquiterpènes de type SB ou de dérivés de celui-ci contenus dans ladite cellule et/ou dans le milieu de culture.

Dans un mode de réalisation particulier, les sesquiterpènes de type SB produits sont récoltés à l'étape c). Dans un mode de réalisation préféré, les sesquiterpènes de type SB produits sont sélectionnés parmi l'alpha-santalène, l'épi-beta-santalène, l'alpha-bergamotène, le beta-bergamotène, et l'endo-beta-bergamotène. Dans un autre mode de réalisation particulier, les sesquiterpènes de type SB produits sont les produits de départ pour obtenir d'autres composés d'intérêt comme l'alpha-santalol. Dans un mode de réalisation préféré, les dérivés de sesquiterpènes de type SB sont sélectionnés parmi l'alpha-santalol, l'épi-beta-santalol, le cis-alpha-bergamotol, le trans-alpha-bergamotol et l'endo-beta-bergamotol.

Dans un mode de réalisation particulier, la présente invention concerne une méthode de production de sesquiterpènes de type SB ou de dérivés de ceux-ci à partir de *Z,Z-*FPP dans une cellule ayant une source de DMAP et IPPP, comprenant :
a) la fourniture d'une cellule recombinante comprenant un premier gène hétérologue codant pour une zFPS selon la présente invention et un deuxième gène hétérologue codant pour une SB synthase selon la présente invention ;
b) la culture de la cellule dans des conditions appropriées pour l'expression desdits premier et deuxième gènes ; et,
c) facultativement, la récolte de sesquiterpènes de type SB ou de dérivés de celui-ci contenus dans ladite cellule et/ou dans le milieu de culture.

Dans un mode de réalisation préféré, ladite cellule produit du *Z,Z*-FPP. Dans un autre mode de réalisation, le *Z,Z-*FPP est fourni à la cellule.

Dans un mode de réalisation particulier, les deux cassettes d'expression sont portées par une même construction. Dans un autre mode de réalisation, les deux cassettes d'expression sont portées par deux constructions distinctes.

La description décrit une protéine isolée ou recombinante ayant une activité *Z,Z-*FPP synthase. En particulier, elle concerne un polypeptide isolé ou recombinant ayant une activité de *Z,Z-*FPP synthase et dont la séquence présente au moins 80%, 85%, 90%, 95%, 97%, 98% ou 99% d'identité avec la SEQ ID No 2. De préférence, le polypeptide présente au moins 95% d'identité avec la SEQ ID No 2. Dans un mode de réalisation particulier, le polypeptide comprend ou consiste en la séquence SEQ ID No 2. Ce polypeptide peut également comprendre une séquence additionnelle permettant de faciliter la purification de l'enzyme, par exemple une séquence étiquette comprenant plusieurs acides aminés histidine consécutifs.

La description décrit également un acide nucléique isolé comprenant une séquence nucléotidique codant un polypeptide ayant une activité de *Z,Z-*FPP synthase et dont la séquence présente au moins 80%, 85%, 90%, 95%, 97%, 98% ou 99% d'identité avec la SEQ ID No 2 ou une séquence capable de s'hybrider à celui-ci dans des conditions stringentes. Un exemple d'acide nucléique est l'acide nucléique comprenant ou consistant en une séquence présentant au moins 80%, 85%, 90%, 95%, 97%, 98% ou 99% d'identité avec la SEQ ID No 1 ou une séquence complémentaire de celle-ci. La description décrit un acide nucléique isolé capable de s'hybrider dans des conditions de forte stringence à un acide nucléique qui code un polypeptide ayant une activité de *Z,Z-*FPP synthase et la séquence dudit polypeptide présentant au moins 95% ou 98% d'identité avec la SEQ ID No 2. Dans un mode de réalisation particulier, l'acide nucléique comprend ou consiste en la séquence SEQ ID No 1. La présente invention concerne également une cassette d'expression, un vecteur, une cellule hôte ou un organisme transgénique comprenant un acide nucléique hétérologue selon la présente invention.

Les acides nucléiques peuvent être des ADN génomiques, des ADN complémentaires (ADNc) ou des ADN synthétiques. Ils peuvent être sous forme simple chaîne ou en duplexe ou un mélange des deux. Dans le cadre de la présente invention, les acides nucléiques transcrits sont de préférence des ADNc dénués d'introns. Les acides nucléiques transcrits peuvent être des molécules synthétiques ou semi-synthétiques, recombinantes, éventuellement amplifiées ou clonées dans des vecteurs, modifiées chimiquement ou comprenant des bases non naturelles. Il s'agit typiquement de molécules d'ADN isolées, synthétisées par des techniques recombinantes bien connues en soi de l'homme du métier.

La présente invention concerne également un polypeptide isolé ou recombinant ayant une activité de SB synthase et dont la séquence présente au moins 80%, 85%, 90%, 95%, 97%, 98% ou 99% d'identité avec la SEQ ID No 4 . Dans un mode de réalisation particulier, le polypeptide comprend ou consiste en la séquence SEQ ID No 4. Dans ce cas, la présente invention concerne un polypeptide isolé ou recombinant ayant une activité de SB synthase et dont la séquence présente au moins 95% d'identité avec la SEQ ID No 4. Ce polypeptide peut également comprendre une séquence additionnelle permettant de faciliter la purification de l'enzyme, par exemple une séquence étiquette comprenant plusieurs acides aminés histidine consécutifs.

La description décrit également un acide nucléique isolé comprenant une séquence nucléotidique codant un polypeptide ayant une activité de SB synthase et dont la séquence présente au moins 80%, 85%, 90%, 95%, 97%, 98% ou 99% d'identité avec la SEQ ID No 4 ou une séquence capable de s'hybrider à celui-ci dans des conditions stringentes. Un exemple d'acide nucléique est l'acide nucléique comprenant ou consistant en une séquence présentant au moins 80%, 85%, 90%, 95%, 97%, 98% ou 99%d'identité avec la SEQ ID No 3 ou une séquence complémentaire de celle-ci. La description décrit un acide nucléique isolé capable de s'hybrider dans des conditions de forte stringence à un acide nucléique qui code un polypeptide ayant une activité de SB synthase et la séquence dudit polypeptide présentant au moins 95% ou 98% d'identité avec la SEQ ID No 4. Dans un mode de réalisation particulier, l'acide nucléique comprend ou consiste en la séquence SEQ ID No 3. La présente invention concerne également une cassette d'expression, un vecteur, une cellule hôte ou un organisme transgénique comprenant un acide nucléique hétérologue selon la présente invention.

La présente invention concerne tout particulièrement un vecteur, une cellule hôte ou un organisme transgénique comprenant un acide nucléique comprenant une séquence hétérologue codant un polypeptide ayant une activité de SB synthase et dont la séquence présente au moins 80%, 85%, 90%, 95%, 97%, 98% ou 99% d'identité avec la SEQ ID No 4 et un acide nucléique comprenant une séquence hétérologue codant un polypeptide ayant une activité de *Z,Z*-FPP synthase et dont la séquence présente au moins 80%, 85%, 90%, 95%, 97%, 98% ou 99% d'identité avec la SEQ ID No 2. Dans un mode de réalisation particulier de l'invention, l'organisme transgénique est une plante, et en particulier une plante à trichomes.

La présente invention concerne également un vecteur, une cellule hôte ou un organisme transgénique comprenant un acide nucléique comprenant une séquence hétérologue codant un polypeptide ayant une activité de SB synthase et dont la séquence présente au moins 80%, 85%, 90%, 95%, 97%, 98% ou 99% d'identité avec la SEQ ID No 4.

La présente invention concerne en outre un vecteur, une cellule hôte ou un organisme transgénique comprenant un acide nucléique comprenant une séquence hétérologue codant un polypeptide ayant une activité de *Z,Z-*FPP synthase, de préférence dont la séquence présente au moins 80%, 85%, 90%, 95%, 97%, 98% ou 99% d'identité avec la SEQ ID No 2.

La description décrit une méthode de production d'un mélange de sesquiterpènes de type SB ou de dérivés de ceux-ci à partir d'IPP et de DMAP comprenant la mise en contact d'IPP et de DMAP avec une *Z,Z-*FPP synthase recombinante ou purifiée présentant au moins 80%, 85%, 90%, 95%, 97%, 98% ou 99% d'identité avec la SEQ ID No 3 et une SB synthase recombinante ou purifiée présentant au moins 80%, 85%, 90%, 95%, 97%, 98% ou 99% d'identité avec la SEQ ID No 4 dans des conditions appropriées et la récolte d'un mélange de sesquiterpène de type SB ou de dérivés de celui-ci obtenus. L'invention concerne l'utilisation d'une *Z,Z-*FPP synthase recombinante ou purifiée présentant au moins 80%, 85%, 90%, 95%, 97%, 98% ou 99% d'identité avec la SEQ ID No 2 et d'une SB synthase recombinante ou purifiée présentant au moins 80%, 85%, 90%, 95%, 97%, 98% ou 99% d'identité avec la SEQ ID No 4 pour la préparation d'un mélange de sesquiterpène de type SB ou de dérivés de celui-ci à partir de d'IPP et de DMAP.

La description décrit une méthode de production de *Z,Z*-FPP ou de dérivés de celui-ci à partir d'IPP et de DMAP comprenant la mise en contact d'IPP et de DMAP avec une *Z,Z-*FPP synthase recombinante ou purifiée présentant au moins 80%, 85%, 90%, 95%, 97%, 98% ou 99% d'identité avec la SEQ ID No 2 dans des conditions appropriées et la récolte du *Z,Z-*FPP ou de dérivés de celui-ci obtenus. Facultativement, la méthode comprend en outre l'incubation du *Z,Z*-FPP obtenu avec une phosphatase alkaline d'intestin de veau et la récolte du *Z,Z*-farnesol. La présente invention concerne l'utilisation d'une *Z,Z-*FPP synthase recombinante ou purifiée présentant au moins 80%, 85%, 90%, 95%, 97%, 98% ou 99% d'identité avec la SEQ ID No 2 pour la préparation de *Z,Z*-FPP ou de dérivés de celui-ci à partir d'IPP et de DMAP.

La description décrit une méthode de production d'un mélange de sesquiterpènes de classe II ou de dérivés de ceux-ci à partir de *Z,Z*-FPP comprenant la mise en contact du *Z,Z-*FPP avec une SB synthase recombinante ou purifiée présentant au moins 80%, 85%, 90%, 95%, 97%, 98% ou 99% d'identité avec la SEQ ID No 4 dans des conditions appropriées et la récolte d'un mélange de sesquiterpène de type SB ou de dérivés de ceux-ci obtenus. L'invention concerne l'utilisation d'une SB synthase recombinante ou purifiée présentant au moins 80%, 85%, 90%, 95%, 97%, 98% ou 99% d'identité avec la SEQ ID No 4 pour la préparation d'un mélange de sesquiterpène de classe II ou de dérivés de ceux-ci à partir de *Z,Z-*FPP.

De manière générale, une cassette d'expression comprend tous les éléments nécessaires à la transcription et traduction du gène en une protéine. Notamment, elle comprend un promoteur, éventuellement un amplificateur, un terminateur de transcription et les éléments permettant la traduction.

Le promoteur est adapté à la cellule hôte. Par exemple, si la cellule est procaryote, le promoteur peut être sélectionné parmi les promoteurs suivants : Lacl, LacZ, pLacT, ptac, pARA, pBAD, les promoteurs d'ARN polymérase de bactériophage T3 or T7, le promoteur de la polyhèdrine, le promoteur PR ou PL du phage lambda. Si la cellule est eucaryote et animale, le promoteur peut être sélectionné parmi les promoteurs suivants : promoteur précoce du cytomegalovirus (CMV), promoteur de la thymidine kinase du virus de l'herpes simple (herpes simplex virus, HSV), promoteur précoce ou tardif du virus simian 40 (SV40), le promoteur de la métallothionéine-L de souris, et les régions LTR (long terminal repeat) de certains rétrovirus. De manière générale, pour le choix d'un promoteur adapté, l'homme du métier pourra avantageusement se référer à l'ouvrage de Sambrook et Russell (2000) ou encore aux techniques décrites dans l'ouvrage d'Ausubel *et al.* (2006)

Le vecteur peut être un plasmide, un phage, un phagemide, un cosmide, un virus, un YAC, un BAC, un plasmide pTi d'*Agrobacterium,* etc. Le vecteur peut comprendre de préférence un ou plusieurs éléments sélectionnés parmi une origine de réplication, un site de clonage multiple et un marqueur. Le marqueur peut être un gène rapporteur qui engendre un signal détectable. Le signal détectable peut être un signal fluorescent, une coloration, une émission de lumière. Le marqueur pourra donc être GFP, EGFP, DsRed, la beta-galactosidase, la beta-glucosidase, la luciférase, etc. Le marqueur est de préférence un marqueur de sélection procurant à la cellule une résistance à un antibiotique ou un herbicide. Par exemple, le gène peut être un gène de résistance à la kanamycine, néomycine, etc. Dans un mode de réalisation préféré, le vecteur est un plasmide. Des exemples de vecteurs procaryotes figurent dans la liste suivante qui n'est pas exhaustive : pQE70, pQE60, pQE-9 (Qiagen), pbs, pD10, phagescript, psiX174, pbluescript SK, pbsks, pNH8A, pNH16A, pNH18A, pNH46A (Stratagene); ptrc99a, pKK223-3, pKK233-3, pDR540, pBR322, et pRIT5 (Pharmacia), pET (Novagen) et pQE-30 (QIAGEN). Des exemples de vecteurs eucaryotes figurent dans la liste suivante qui n'est pas exhaustive : pWLNEO, pSV2CAT, pPICZ, pcDNA3.1 (+) Hyg (Invitrogen), pOG44, pXT1, pSG (Stratagene); pSVK3, pBPV, pCI-neo (Stratagene), pMSG, pSVL (Pharmacia);. Les vecteurs viraux peuvent être de manière non-exhaustive des adénovirus, des AAV, des HSV, des lentivirus, etc. De préférence, le vecteur d'expression est un plasmide ou un vecteur viral.

La cellule hôte peut être un procaryote, par exemple *Escherichia coli, Bacillus subtilis, Streptomyces* sp, et *Pseudomonas* sp ou un eucaryote. L'eucaryote peut être un eucaryote inférieur comme une levure (par exemple, *Saccharomyces cerevisiae*) ou un champignon filamenteux (par exemple du genre *Aspergillus*) ou un eucaryote supérieur comme une cellule d'insecte, de mammifère ou de plante. La cellule peut être une cellule mammifère, par exemple une cellule COS, CHO (US 4,889,803 ; US 5,047,335). Dans un mode de réalisation particulier, la cellule est non-humaine et non-embryonnaire. La cellule peut être isolée, par exemple dans un milieu de culture. Les cellules peuvent également être comprises dans des organismes, par exemple des animaux non-humains transgéniques ou des plantes transgéniques.

La présente invention concerne donc une méthode de production de *Z,Z-*FPP ou de dérivés de celui-ci à partir d'IPP et de DMAP dans une cellule ayant une source d'IPP et de DMAP, comprenant :
a) l'introduction dans ladite cellule d'une construction portant une cassette d'expression comprenant un gène codant pour une *Z,Z-*FPP synthase selon la présente invention ;
b) la culture de la cellule transformée dans des conditions appropriées pour l'expression du gène ; et,
c) facultativement, la récolte du *Z,Z-*FPP ou de dérivés de celui-ci contenus dans ladite cellule et/ou dans le milieu de culture.

Dans un mode de réalisation particulier, le *Z,Z-*FPP produit est récolté à l'étape c). Dans un autre mode de réalisation particulier, le *Z,Z-*FPP produit est le produit de départ pour obtenir un autre composé d'intérêt comme le *Z,Z-*farnesol. Par exemple, le *Z,Z-*FPP peut être transformé en *Z,Z*-farnesol par une phosphatase. Ainsi, la méthode peut comprendre en outre à l'étape a) l'introduction d'une construction portant une cassette d'expression comprenant un gène codant pour une phosphatase, permettant ainsi la récolte de *Z,Z-*farnesol à l'étape c). La description décrit donc une méthode de production de *Z,Z-*FPP ou de dérivés de celui-ci à partir d'IPP et de DMAP dans une cellule recombinante ayant une source d'IPP et de DMAP et comprenant un gène hétérologue codant pour une *Z,Z-*FPP synthase selon la présente description. La présente invention concerne ainsi une méthode de production de *Z,Z-*FPP dans une cellule recombinante ayant une source de DMAP et IPPP, comprenant :
a) la fourniture d'une cellule recombinante comprenant un gène hétérologue codant pour une zFPS selon la présente invention ;
b) la culture de la cellule dans des conditions appropriées pour l'expression dudit gène ; et,
c) facultativement, la récolte du *Z,Z-*FPP ou de dérivés de celui-ci contenus dans ladite cellule et/ou dans le milieu de culture.

La description décrit donc une méthode de production d'un mélange de sesquiterpènes de type SB ou de dérivés de ceux-ci à partir de *Z,Z-*FPP dans une cellule ayant une source de *Z,Z*-FPP, comprenant :
a) l'introduction dans ladite cellule d'une construction portant une cassette d'expression comprenant un gène codant pour une *SB* synthase selon la présente description ;
b) la culture de la cellule transformée dans des conditions appropriées pour l'expression du gène ; et,
c) facultativement, la récolte d'un mélange de sesquiterpène de type SB ou de dérivés de celui-ci contenus dans ladite cellule et/ou dans le milieu de culture.

Dans un mode de réalisation particulier, le mélange de sesquiterpène de type SB produit est récolté à l'étape c). Dans un autre mode de réalisation particulier, le mélange de sesquiterpènes de type SB produit est le produit de départ pour obtenir d'autres composés d'intérêt comme l'alpha-santalol. La description décrit concerne donc une méthode de production d'un mélange de sesquiterpène de type SB ou de dérivés de ceux-ci à partir de *Z,Z*-FPP dans une cellule recombinante ayant une source de *Z,Z*-FPP et comprenant un gène hétérologue codant pour une SB synthase selon la présente description. La présente invention concerne ainsi une méthode de production de sesquiterpènes de type SB ou de dérivés de ceux-ci à partir de *Z,Z-*FPP dans une cellule recombinante ayant une source de *Z,Z*-FPP, comprenant :
a) la fourniture d'une cellule recombinante comprenant un gène hétérologue codant pour une SB synthase selon la présente invention ;
b) la culture de la cellule dans des conditions appropriées pour l'expression dudit gène ; et,
c) facultativement, la récolte de sesquiterpènes de type SB ou de dérivés de celui-ci contenus dans ladite cellule et/ou dans le milieu de culture.

La cellule peut également être partie d'un organisme multicellulaire, par exemple une plante ou un animal non-humain. Dans ce cas, la description décrit une méthode de préparation d'un organisme multicellulaire comprenant :
a) l'introduction dans une cellule de l'organisme d'une construction portant une cassette d'expression comprenant un premier gène codant pour une *Z,Z-*FPP synthase selon la présente description et d'une construction portant une cassette d'expression comprenant un deuxième gène codant pour une SB synthase selon la présente description ; et,
b) la reconstitution d'un organisme à partir de ladite cellule et la sélection des organismes transgéniques exprimant les deux gènes introduits.

Dans un mode de réalisation, l'organisme est un animal non-humain. Par exemple, l'organisme peut être une souris, un rat, un cobaye, un lapin, etc. Dans un autre mode de réalisation préféré, l'organisme est une plante, de préférence une plante à trichomes glanduleux.

La description décrit donc une méthode de production d'un mélange de sesquiterpènes de type SB comprenant la fourniture d'un organisme multicellulaire transgénique exprimant une *Z,Z-*FPP synthase selon la présente description et une SB synthase selon la présente description et la récolte d'un mélange de sesquiterpènes de type SB produit ou de dérivés de ceux-ci dans lesdits organismes transgéniques.

La méthode permet de faire produire un mélange de sesquiterpène de type SB à des organismes ne produisant pas ces composés ou d'augmenter la quantité d'un mélange de sesquiterpène de type SB produit par des organismes le produisant déjà.

La description décrit une méthode de préparation d'un organisme multicellulaire comprenant :
a) l'introduction dans une cellule de l'organisme d'une construction portant une cassette d'expression comprenant un gène codant pour une *Z,Z-*FPP synthase selon la présente description; et
b) la reconstitution d'un organisme à partir de ladite cellule et la sélection des organismes transgéniques exprimant le gène introduit.

La présente invention concerne également une méthode de production de *Z,Z-*FPP ou de dérivés de celui-ci comprenant comprenant la fourniture d'un organisme multicellulaire transgénique exprimant une *Z,Z-*FPP synthase selon la présente description et la récolte du *Z,Z*-FPP produit ou de dérivés de celui-ci dans lesdits organismes transgéniques.

Dans un mode de réalisation, l'organisme est un animal non-humain. Par exemple, l'organisme peut être une souris, un rat, un cobaye, un lapin, etc. Dans un autre mode de réalisation préféré, l'organisme est une plante, de préférence une plante à trichomes glanduleux. La méthode permet de faire produire du *Z,Z-*FPP à des organismes ne produisant pas ce composé ou d'augmenter la quantité de *Z,Z-*FPP produit par des organismes.

La description décrit une méthode de préparation d'un organisme multicellulaire comprenant :
a) l'introduction dans une cellule de l'organisme d'une construction portant une cassette d'expression comprenant un gène codant pour une SB synthase selon la présente description ; et
b) la reconstitution d'un organisme à partir de ladite cellule et la sélection des organismes transgéniques exprimant le gène introduit.

La description décrit une méthode de production d'un mélange de sesquiterpène de type SB ou de dérivés de ceux-ci comprenant la fourniture d'un organisme multicellulaire transgénique exprimant une SB synthase selon la présente description et la récolte d'un mélange de sesquiterpène de type SB produit ou de dérivés de ceux-ci dans lesdits organismes transgéniques.

Dans un mode de réalisation, l'organisme est un animal non-humain. Par exemple, l'organisme peut être une souris, un rat, un cobaye, un lapin, etc. Dans un autre mode de réalisation préféré, l'organisme est une plante, de préférence une plante à trichomes sécréteurs. La méthode permet de faire produire un mélange de sesquiterpènes de type SB à des organismes ne produisant pas ce composé ou d'augmenter la quantité d'un mélange de sesquiterpènes de type SB produit par des organismes.

L'invention est notamment applicable à toutes les plantes de familles possédant des trichomes glanduleux, par exemple les Astéracées (tournesol, etc.), Solanacées (tomate, tabac, pomme de terre, poivron, aubergine, etc.), Cannabacées (ex *Cannabis sativa*) et Lamiacées (menthe, basilic, lavande, thym, etc.). Elle est particulièrement adaptée aux plantes de la famille des Solanacées, telles que par exemple des genres Nicotiana, Solanum, Capsicum, Petunia, Datura, Atropa, etc., et en particulier des genres *Solanum* et Nicotiana comme par exemple la tomate cultivée (*Solanum lycopersicum*)*,* la tomate sauvage *Solanum habrochaites,* le tabac cultivé (*Nicotiana tabacum*)*,* et le tabac sylvatique *(Nicotiana sylvestris*)*.* De manière non limitative, l'invention peut s'appliquer aux plantes des genres suivants : Populus, Nicotiana, Cannabis, Pharbitis, Apteria, Psychotria, Mercurialis, Chrysanthemum, Polypodium, Pelargonium, Mimulus, Matricaria, Monarda, Solanum, Achillea, Valeriana, Ocimum, Medicago, Aesculus, Plumbago, Pityrogramma, Phacelia, Avicennia, Tamarix, Frankenia, Limonium, Foeniculum, Thymus, Salvia, Kadsura, Beyeria, Humulus, Mentha, Artemisia, Nepta, Geraea, Pogostemon, Majorana, Cleome, Cnicus, Parthenium, Ricinocarpos, Hymennaea, Larrea, Primula, Phacelia, Dryopteris, Plectranthus, Cypripedium, Petunia, Datura, Mucuna, Ricinus, Hypericum, Myoporum, Acacia, Diplopeltis, Dodonaea, Halgania, Cyanostegia, Prostanthera, Anthocercis, Olearia, Viscaria. De préférence, la plante est une plante de la famille des Asteracées, Cannabacées, Solanacées ou Lamiacées. Dans un mode de réalisation encore plus préféré, la plante appartient aux genres Solanum ou Nicotiana, de préférence *Solanum esculentum, Solanum habrochaites, Nicotiana tabacum* ou *Nicotiana sylvestris.*

Dans ce mode de réalisation, les gènes sont placés sous le contrôle d'un promoteur permettant une expression, de préférence spécifique, dans les trichomes de la plante. De tels promoteurs existent et sont connus par l'homme du métier (Tissier *et al.,* 2004).

Au sens de l'invention, on entend par promoteur "spécifique" un promoteur principalement actif dans un tissu ou un groupe cellulaire donné. Il est entendu qu'une expression résiduelle, généralement plus faible, dans d'autres tissus ou cellules ne peut être entièrement exclue. Une caractéristique particulière de l'invention réside dans la capacité de construire des promoteurs spécifiques des cellules sécrétrices des trichomes glanduleux, permettant une modification de la composition des sécrétions foliaires de la plante, et notamment d'y exprimer les gènes de la présente invention permettant de préparer le mélange de sesquiterpène de type SB. Ainsi, le *Z,Z*-FPP, et le mélange de sesquiterpènes de type SB ou des dérivés de ceux-ci peuvent être récoltés au niveau des trichomes de ces plantes, en particulier dans l'exsudat des trichomes par extraction avec un solvant ou encore par distillation.

Par exemple, il a été montré qu'une séquence régulatrice de 1852 pb, située en amont de l'ATG du gène CYP71D16, permet de diriger l'expression du gène rapporteur *uidA* spécifiquement dans les cellules sécrétrices de trichomes de tabac (demande US2003/0100050 A1, Wagner et al., 2003). D'autre part, plusieurs séquences promotrices extraites de différentes espèces ont été identifiées comme étant capables de diriger l'expression d'un gène hétérologue dans les trichomes de tabac.

| Gène Abréviation | Nom du gène | Plantes | Références |
|---|---|---|---|
| LTP3 | Lipid transfer protein | Cotton | Liu et al., 2000, BBA, 1487:106111 |
| LTP6 | | Cotton | Hsu et al., 1999, Plant science, 143:6370 |
| wax9D | | B. oleracea | Pyee and Kolattukudy, 1995, Plant J. 7:4559 |
| LTP1 | | Arabidopsis | Thoma et al., 1994, Plant Physiol. 105 3545 |
| CYC71D16 | CBTol hydroxylase | Tobacco | Wang et al., 2002, J.of Exp. Bot. 18911897 |

Dans un mode de réalisation préféré de la présente invention, le promoteur utilisé dans la cassette est dérivé des gènes NsTPS-02a, 02b, 03, et 04 de l'espèce *Nicotiana sylvestris* possédant une forte similarité de séquence avec CYC-2 (CBT-ol cyclase; NID : AF401234). Ces séquences promotrices sont plus amplement décrites dans la demande de brevet WO2006040479 (Tissier et al., 2004).

Parmi les séquences terminateurs préférées, on peut citer le terminateur NOS (Bevan *et al.*, 1983), et le terminateur du gène d'histone (EPO 633 317).

Dans un mode de réalisation particulier, la cassette d'expression peut comprendre une séquence permettant d'augmenter l'expression (« amplificateur »), par exemple certains éléments du promoteur CaMV35S et de gènes de l'octopine synthase (US 5 290 924). De préférence, les éléments activateurs du promoteur CaMV35S sont utilisés.

L'introduction des constructions portant un ou les deux gènes de l'invention dans une cellule ou un tissu végétal, y compris une graine ou plante, peut être réalisée par toute technique connue de l'homme du métier, y compris sous forme épisomale ou chromosomique. Les techniques de transgenèse végétale sont bien connues dans le domaine, et comprennent par exemple l'utilisation de la bactérie *Agrobacterium tumefaciens*, l'électroporation, des techniques biolistiques, la transfection par un vecteur viral notamment, et toute autre technique connue de l'homme du métier.

Une technique communément utilisée repose sur l'utilisation de la bactérie *Agrobacterium tumefaciens,* qui consiste essentiellement à introduire la construction d'intérêt (acide nucléique, cassette, vecteur, etc.) dans la bactérie *A. tumefaciens*, puis à mettre en contact cette bactérie transformée avec des disques de feuilles de la plante choisie. L'introduction de la cassette d'expression dans la bactérie est typiquement réalisée en utilisant comme vecteur le plasmide Ti (ou T-DNA), qui peut être transféré dans la bactérie par exemple par choc thermique ou par électroporation. L'incubation de la bactérie transformée avec les disques foliaires permet d'obtenir le transfert du plasmide Ti dans le génome des cellules des disques. Ceux-ci peuvent éventuellement être cultivés dans des conditions appropriées pour reconstituer une plante transgénique dont les cellules comprennent la construction de l'invention. Pour plus de détails ou des variantes de mise en oeuvre de la technique de transformation par *A. tumefaciens*, on peut se référer par exemple à Horsch *et al.* (1985) ou Hooykaas and Schilperoort (1992).

Ainsi, dans un mode de réalisation particulier, la cassette d'expression ainsi constituée est insérée entre les bordures gauche et droite de l'ADN de transfert (T-DNA) d'un plasmide Ti désarmé pour le transfert dans des cellules végétales par *Agrobacterium tumefaciens.* Le T-DNA comprend également un gène dont l'expression confère une résistance à un antibiotique ou un herbicide et qui permet la sélection des transformants.

Une fois régénérées, les plantes transgéniques peuvent être testées pour l'expression hétérologue des gènes de la présente invention ou la production d'un mélange de sesquiterpènes de type SB, de *Z,Z-*FPP ou de *Z,Z*-farnesol dans les trichomes. Ceci peut être réalisé en récoltant l'exsudat des feuilles et en testant la présence du produit d'intérêt dans cet exsudat. L'analyse des volatiles émis par la plante peut également permettre d'identifier les dits composés. Ceci peut également être fait en analysant la présence d'une ou des enzymes hétérologues de la présente invention dans les feuilles et, plus particulièrement, dans les cellules de trichome (par exemple en analysant les ARNm ou l'ADN génomique au moyen de sondes ou d'amorces spécifiques). Les plantes peuvent éventuellement être sélectionnées, croisées, traitées, etc. pour obtenir des plantes présentant des niveaux d'expression améliorés.

Un autre objet de l'invention réside également dans une plante ou graine comprenant un acide nucléique, une cassette d'expression ou un vecteur tels que définis précédemment.

Un autre objet de l'invention concerne l'utilisation d'un acide nucléique de la présente invention comme marqueurs moléculaires pour permettre d'introduire les séquences génomiques correspondantes de *S*. *habrochaites* dans d'autres espèces ou variétés de tomate cultivée *(S. lycopersicum)* sexuellement compatibles. En effet, ces marqueurs permettent de contrôler l'introduction des séquences génomiques correspondantes de *S*. *habrochaites* dans d'autres espèces ou variétés de tomate cultivée *(S. lycopersicum)* sexuellement compatibles et ainsi, par exemple, d'identifier et de sélectionner les individus issus d'un croisement entre l'espèce sauvage et une espèce cultivée dans lesquels l'introgression des acides nucléiques de la présente invention a eu lieu. L'utilisation des acides nucléiques de la présente invention comme marqueurs moléculaires peut être réalisée par toute technique connue de l'homme du métier. Par exemple, une technique communément utilisée repose sur l'identification d'un polymorphisme de bande (RFLP) entre deux génomes digérés par différentes enzymes de restriction par hybridation moléculaire avec les acides nucléiques de la présente invention. Un autre exemple, consiste à rechercher un polymorphisme en utilisant la technique de PCR pour amplifier sur les génomes à comparer tout ou partie d'un acide nucléique de la présente invention à partir d'amorces complémentaires de l'acide nucléique de la présente invention pour identifier après amplification PCR un polymorphisme de taille entre les deux génomes. Le polymorphisme peut également être révélé après digestion desdits produits PCR par une ou des enzymes de restriction qui feront apparaître des fragments de taille différente entre les génomes comparés.

Une fois le polymorphisme identifié, cette information est utilisée, par exemple, pour identifier les individus d'une population F2 issus d'un croisement entre *S*. *habrochaites* et une variété de tomate dans laquelle l'introgression des acides nucléiques de la présente invention est souhaitée.

Un autre objet de l'invention concerne l'introduction des séquences génomiques de *S*. *habrochaites* codant pour la zFPS et la SB synthase dans des espèces non compatibles sexuellement par fusion de cellules, notamment de protoplastes. Les techniques de fusion de protoplastes sont connus de l'homme du métier (Zimmermann *et al.*, 1981, Bates *et al.*, 1983), et permettent de créer des cellules hybrides contenant des chromosomes appartenant aux deux espèces fusionnées. Lors de la fusion des chromosomes ou fragments de chromosome des deux espèces sont éliminés. Les cellules hybrides ayant conservé le ou les fragments chromosomiques contenant les gènes de la présente invention peuvent alors être sélectionnées par PCR en utilisant des amorces spécifiques du gène codant pour la zFPS (SEQ ID N°1) ou de celui codant pour la SB synthase (SEQ ID N°2) ou des deux à la fois.

La présente invention concerne enfin une cellule ou un organisme transgénique non-humain, caractérisé en ce que la voie de synthèse d'un mélange de sesquiterpène de type SB est bloquée par inactivation du gène codant pour une *Z,Z*-FPP synthase selon la présente invention ou du gène codant pour une SB synthase selon la présente invention ou des deux gènes. L'expression de ces gènes peut être bloquée par de nombreuses techniques disponibles et connues par l'homme du métier. Les gènes peuvent être délétés, mutés (par exemple par mutagenèse chimique par Ethyl Methane Sulfonate ou par rayonnements ionisants) ou interrompus (mutagenèse insertionnelle). Par ailleurs, le blocage de l'expression de ces gènes peut également être réalisé par extinction de gènes en exprimant un transcrit inhibiteur. Le transcrit inhibiteur est un ARN qui peut se présenter sous la forme d'un ARN double brin, d'un ARN antisens, d'un ribozyme, d'un ARN capable de former une triple hélice, et qui a une certaine complémentarité ou spécificité avec le transcrit du gène à bloquer.

La description décrit également un acide nucléique capable de diminuer ou de supprimer l'expression d'un gène codant une *Z,Z*-FPP synthase. En effet, un ARN inhibiteur qui peut se présenter sous la forme d'un ARN double brin, d'un ARN antisens, d'un ribozyme, d'un ARN capable de former une triple hélice, et qui a une certaine complémentarité ou spécificité avec le gène codant *Z,Z*-FPP synthase peut être préparé sur la base de l'enseignement de la présente invention. Ainsi, la description décrit un ARN inhibiteur du gène codant une *Z,Z*-FPP synthase comprenant au moins 21, 30, 40, 50, 60, 70, 80, 90 ou 100 nucléotides consécutifs de la SEQ ID No 1 ou d'une séquence complémentaire de celle-ci. De même, elle décrit l'utilisation d'un polynucléotide comprenant au moins 21, 30, 40, 50, 60, 70, 80, 90 ou 100 nucléotides consécutifs de la SEQ ID No 1 ou d'une séquence complémentaire de celle-ci pour inhiber l'expression du gène codant une *Z,Z-*FPP synthase. L'inhibition de cette enzyme peut être utile pour stopper ou ralentir la voie de synthèse du mélange de sesquiterpène de type SB et rendre l'IPP et le DMAP disponible pour une autre voie de synthèse d'intérêt nécessitant un apport en *Z,Z*-FPP. Par exemple, l'inhibition de cette enzyme peut permettre d'augmenter le taux de production germacrene B par les cellules glandulaires des trichomes de feuilles de tomate. La présente invention concerne donc des cellules ou des organismes transgéniques dans lesquels le gène codant une *Z,Z*-FPP synthase a été inactivé. L'inactivation peut être effectuée par des techniques d'ARN interférent, de délétion de gène, de mutation chimique ou d'inactivation par recombinaison homologue. Ainsi le taux de *Z,Z*-FPP ou du mélange de sesquiterpène de type SB peut être diminué dans ces cellules ou organismes transgéniques.

En outre, la description décrit également un acide nucléique capable de diminuer ou de supprimer l'expression d'un gène codant une SB synthase. En effet, un ARN inhibiteur qui peut se présenter sous la forme d'un ARN double brin, d'un ARN antisens, d'un ribozyme, d'un ARN capable de former une triple hélice, et qui a une certaine complémentarité ou spécificité avec le gène codant une SB synthase peut être préparé sur la base de l'enseignement de la présente invention. Ainsi, description décrit un ARN inhibiteur du gène codant une SB synthase comprenant au moins 30, 40, 50, 60, 70, 80, 90 ou 100 nucléotides consécutifs de la SEQ ID No 3. De même, elle concerne l'utilisation d'un polynucléotide comprenant au moins 21, 30, 40, 50, 60, 70, 80, 90 ou 100 nucléotides consécutifs de la SEQ ID No 3 pour inhiber l'expression du gène codant une SB synthase. L'inhibition de cette enzyme peut être utile pour stopper la voie de synthèse d'un mélange de sesquiterpène de type SB et rendre le *Z,Z*-FPP disponible pour une autre voie de synthèse d'intérêt nécessitant un apport en ce composé, par exemple pour préparer des dérivés du *Z,Z*-FPP, par exemple, le *Z,Z* farnesol, ou d'autres sesquiterpènes qui sont le produit de synthases utilisant le *Z,Z*-FPP comme substrat.

La présente invention concerne donc des cellules ou des organismes transgéniques dans lesquels le gène codant une SB synthase est inactivé. L'inactivation peut être effectuée par des techniques d'ARN interférent, de délétion de gène, de mutation chimique ou d'inactivation par recombinaison homologue. Ainsi le taux du mélange de sesquiterpènes de type SB peut être diminué dans ces cellules ou organismes transgéniques.

La description décrit également une méthode d'identification ou de clonage d'autres gènes codant une *Z,Z*-FPP synthase ou une SB synthase provenant d'une autre espèce dans laquelle une sonde présentant au moins 15, 30, 50, 75, 100, 200 ou 300 nucléotides consécutifs de la séquence SEQ ID No 1 ou 3, respectivement, est préparée et utilisée pour identifier ou sélectionner dans un échantillon une séquence nucléotidique capable de s'hybrider à ladite sonde. L'échantillon peut être par exemple une banque génomique ou de cDNA provenant d'un ou plusieurs organismes. La méthode peut comprendre une étape supplémentaire de caractérisation de la séquence identifiée ou sélectionnée. Cette étape supplémentaire peut comprendre le clonage, et/ou le séquençage, et/ou un alignement de séquence, et/ou un test d'activité enzymatique.

D'autres aspects et avantages de la présente invention apparaîtront à la lecture des exemples qui suivent, qui doivent être considérés comme illustratifs et non limitatifs.

### Exemples

### Synthèse d'ADNc de feuilles de tomate (S. habrochaites)

Des ARNm ont été extraits à partir de feuilles de tomate (*Solanum habrochaites = L. Hirsutum LA1777*) en utilisant un kit d'extraction d'ARN totaux (RNeasy minikit, Qiagen). Les ADNc correspondants ont été synthétisés par transcription inverse à partir d'une amorce polyT en utilisant une transcriptase inverse (Roche, Cat. N° 03 531 317) dans les conditions réactionnelles préconisées par le fabriquant.

### Clonage des séquences codantes pour la Z,Z-FPP synthase (Sh-zFPS) et la SB synthase (Sh-SBS) de tomate Solanum habrochaites.

Les séquences nucléotidiques codantes et complètes pour les deux gènes *Sh-zFPS* et *Sh-SBS* ont été clonées à partir des informations publiques de séquences de la banque d'EST de trichomes de feuilles de tomate de *S*. *habrochaites* (Van der Hoeven, *et al.*, 2000, unpublished,) obtenu sur le site du SGN (« SOL Genomics Network », http://www.sgn.comell.edu/).

Les séquences de *Sh-zFPS* (SEQ ID N°1) et *Sh-SBS* (SEQ ID N°3) ont été obtenues par PCR à partir des ADNc de feuilles de tomate en utilisant les amorces 5'-ACCATGGGTTCTTTGGTTCTTCAATGTTGGA-3' (SEQ ID No 5) et 5'-ACTCGAGATATGTGTGTCCACCAAAACGTCTATG-3' (SEQ ID No 6) pour la séquence ID N°1 et les amorces 5'-TCCATGGTAGTTGGCTATAGAAGCACAATCA-3' (SEQ ID No 7) et 5'-TCTCGAGCATAAATTCAAATTGAGGGATTAATGA-3' (SEQ ID No 8) pour la séquence ID N°3. Des sites d'enzymes de restriction (NcoI et XhoI) ont été ajoutés à l'extrémité 5' des amorces pour faciliter le clonage du cDNA en aval d'un promoteur. L'amplification par PCR a été réalisée avec 0,5 unité de Taq polymérase (Eurogentec) dans le tampon fourni par le fabriquant. Le produit PCR a été purifié sur une colonne Qiaquick (Qiagen) et cloné par ligation avec une T4 DNA ligase (NEB) dans le vecteur de clonage pGEM-T (Promega). Les clones correspondant à la séquence attendue ont été sélectionnés après séquençage complet des inserts.

La séquence nucléotidique *Sh-zFPS* (SEQ ID N°1) a une longueur de 912 bases et code pour une protéine (SEQ ID N°2) de 303 acides aminés. Un domaine protéique conservé de type *cis* (Z)-isoprényles diphosphate synthases (cis-IPPS) ou également annoté undécaprényle synthase (UPPS) a été identifié « *in silico* » avec le système CCD (NCBI, Marchler-Bauer A, Bryant SH (2004). Les séquences similaires en acide aminé à *Sh*-zFPS ont été recherchées dans la banque de protéine SwissProt et GenBank en utilisant le programme BlastP (Altschul *et al.*, 1997). Les meilleurs pourcentages d'identité ont été obtenus avec des séquences de plantes ayant une homologie avec des undécaprényles diphosphate synthases mais dont les fonctions n'ont pas été démontrées (Tab. 1). Par exemple, *Sh*-zFPS possède un pourcentage d'identité de 42% et 39% avec des UPPS putatives d'*Arabidopis thaliana* BAA97345 et AAO63349 respectivement. On peut noter également que la séquence *Sh*-zFPS possède un très faible degré d'identité (24 %) avec la *Z,E*-FPS de *Micrococcus tuberculosis* (053434).

**Tableau 1 : Séquences présentant le meilleur pourcentage d'identité (% identité) obtenu par recherche d'homologie avec le programme BlastP 2.2.13 (Altschul et al., 1997) contre les banque de SwissProt et de GenBank avec la séquence Sh-zFPS.**

| **Organisme** | **PID** | **Fonction** | **% identité** |
|---|---|---|---|
| Arabidopsis thaliana | BAA97345 | Putative UPPS | 42 |
| Arabidopsis thaliana | AA063349 | Putative UPPS | 39 |
| Arabidopsis thaliana | AAO42764 | Putative UPPS | 39 |
| Anabaena sp. PCC 7120 | DP58563 | Putative UPPS | 37 |
| Oryza sativa | NP_915561 | Putative UPPS | 36 |
| Synechococcus elongatus | Q8DI29 | Putative UPPS | 35 |
| Micrococcus luteus | 082827 | Putative UPPS | 34 |
| Gloeobacer violaceus | Q7NPE7 | Putative UPPS | 32 |
| Micrococcus tuberculosis | P60479 | UPPS | 30 |
| Micrococcus tuberculosis | 053434 | *E,Z*-FPS | 24 |

La séquence nucléotidique *Sh-SBS* (SEQ ID N°3) a une longueur de 2334 bases et code pour une protéine (SEQ ID N°4) de 777 acides aminés. Un domaine protéique conservé de terpène cyclase (cd00684.2) a été identifié « *in silico »* avec le système CCD (NCBI, Marchler-Bauer A, Bryant SH (2004). Les séquences similaires en acide aminé à Sh-SBS ont été recherchées dans la banque de protéine SwissProt et GenBank en utilisant le programme BlastP (Altschul *et al.*, 1997). A l'exception d'une séquence de tabac de fonction inconnue (60% d'identité), les meilleurs homologies ont été obtenues avec des diterpènes synthases de plantes (Tab. 2) avec un pourcentage d'identité d'environ 40 à 45% avec des séquences codant pour des kaurène synthases de différentes plantes (*Arabidopsis thaliana, Cucurbita maxima, Stevia rebaudiana, Oryza sativa*). Sh-SBS possède également des homologies plus faible (26 à 33% d'identité) avec des terpènes synthases de fonction connue comme l'abietadiene synthase et la taxadiène synthase et et l'alpha-bisabolène synthase.

**Tableau 2 : Séquences présentant le meilleur pourcentage d'identité (% identité) obtenu par recherche d'homologie avec le programme BlastP 2.2.13 (Altschul et al., 1997) contre les banques de SwissProt et de GenBank avec la séquence Sh-SBS.**

| **Organisme** | **PID** | **Fonction** | **% identité** |
|---|---|---|---|
| Nicotiana tabacum | AAS98912 | Putative terpene synthase | 60 |
| Arabidopsis thaliana | AAC39443 | Kaurene synthase | 44 |
| Lactuca sativa | BAB12441 | Putative kaurene synthase | 43 |
| Cucurbita maxima | Q39548 | Kaurene synthase | 43 |
| Medicago troncatula | ABE87878 | Putative terpene synthase | 43 |
| Stevia rebaudiana | AAD34294 | Kaurene synthase | 42 |
| Oryza sativa | AAQ72559 | Kaurene synthase | 41 |
| Hordeum vulgare | AAT49066 | Kaurene synthase | 36 |
| Abies grandis | AAC24192 | Alpha-bisabolene synthase | 33 |
| Abies grandis | AAB05407 | Abietadiene synthase | 27 |
| Taxus baccata | 2211347A | Taxadiene synthase | 26 |

### Production des protéines recombinantes Sh-zFPS et Sh-SBS

Les séquences nucléiques codant pour *Sh*-zFPS et *Sh-SBS* (séquences ID N° 1 et 3 respectivement) ont été introduites séparément dans le vecteur d'expression pET-30b (Novagen). Pour permettre la purification des protéines recombinantes, les codons STOP de ces séquences ont été supprimés afin de créer une protéine de fusion avec une queue de six histidines à l'extrémité C-terminale. Les vecteurs *Sh*-zFPS-pET30b et *Sh*-SBS-pET30b ainsi obtenus ont été introduits par électroporation dans des cellules d'*Escherichia coli* modifiées pour l'expression de protéines recombinantes (BL21-CodonPlus, Stratagene). Pour la production des protéines, les cultures (500 ml) de *E*. *coli* sont initiées à 37 °C jusqu'à une densité optique de 0,5 à 600 nm. A ce stade, la température de culture est abaissée à 16 °C et de l'éthanol (1%, v/v) est ajouté dans les milieux. Après une heure d'incubation, l'IPTG est ajouté au milieu à une concentration de 1 mM pour induire l'expression de la protéine recombinante. La culture est alors incubée pour une durée de 18 heures. La culture est arrêtée par centrifugation des cellules à 4 °C. Le culot cellulaire est repris dans un tampon phosphate 200mM. Les cellules sont lysées à la presse de French (ΔP = 19 bar). Les lysats sont centrifugés à 15.000×g. Les sumageants contenant les protéines solubles sont dessalés sur colonne PD10 (Amersham), équilibrés avec un tampon phosphate (pH 7, 50 mM), puis appliqués sur une résine d'affinité nickel-nitrilotriacétique (Ni-NTA, Qiagen). Le protocole d'élution du fabricant est strictement suivi. Les fractions les plus enrichies en protéines Sh-zFPS-6His et Sh-SBS-6His sont identifiées par gel SDS-PAGE, puis dessalées sur PD10 équilibrées avec un tampon (Hepes 50 mM, pH 7.8, KCl 100 mM). Un facteur d'enrichissement d'environ 20 a été obtenu pour les deux protéines par rapport à la fraction protéique brute.

### Test d'activité enzymatique in vitro des protéines recombinantes Sh-zFPS et Sh-SBS

Les tests d'activité sont réalisés dans un tampon ayant la composition suivante : Hepes 50 mM, pH 7.8, KCl 100 mM, MgCl₂ 7.5 mM, glycérol 5% (w/v), et DTT 5 mM. Pour les tests d'activité, 25 ou 50 µg de protéines provenant de la fraction enrichie ont été incubés avec les substrats IPP, DMAPP, GPP, FPP, GGPP (Sigma), seuls ou en combinaison, à une concentration de 65µM chacun à 32 °C pendant 2 heures.

Pour les tests réalisés avec Sh-zFPS-6His, les produits de la réaction sont ensuite déphosphorylés par ajout de 20 unités de phosphatase alkaline d'intestin de veau (New England Biolabs) pendant 1 heure à 37 °C. Les terpènes alcools, produits de la déphosphorylation, sont ensuite extraits par un mélange chloroforme:méthanol:eau (0,5:1:0,4). Les phases aqueuses et organiques sont séparées par ajout d'eau (0,5 vol) et de chloroforme (0,5 vol), et centrifugées (2.000xg). Les phases organiques sont prélevées et séchées sous courant d'azote gazeux, reprises dans du pentane et analysées par chromatographie gazeuse couplée à un spectromètre de masse (GC-MS 5973N, Agilent Technologies). Les terpènes alcools sont identifiés par comparaison de leur spectre de masse avec ceux de la base de données du National Institute of Standards and Technology (NIST), et par superposition des temps de rétention avec des standards de geraniol, farnesol et geranylgeraniol (Fluka).

Pour les tests réalisés avec Sh-SBS-6His, les terpènes produits dans la réaction sont directement extraits (à 3 reprises) des mélanges réactionnels avec du pentane (volume à volume). Les 3 extraits pentanes sont rassemblés, concentrés sous courant d'azote gazeux, et analysés par GC-MS. Les terpènes produits sont identifiés par interrogation de la base de données NIST et comparaison avec un chromatogramme d'extrait d'exsudat de *Solanum habrochaites*.

La protéine recombinante Sh-zFPS-6His a été incubée avec les substrats IPP+DMAPP ou IPP+GPP (cf. tableau 1). Seule la première combinaison (IPP+DMAPP) conduit, après déphosphorylation, à la formation d'un terpène alcool unique de 15 atomes de carbones (C15), dont le spectre de masse permet de l'identifier comme un farnesol par comparaison avec le spectre standard présent dans la base de données NIST (figure 4). Le temps de rétention a été comparé à un standard de farnesol contenant un mélange des 4 isomères du farnesol (*E,E*-farnesol ; *E,Z*-farnesol ; *Z,E* farnesol et *Z,Z*-farnesol). Le famesol produit par Sh-zFPS-6His a un temps de rétention identique à celui du *Z,Z*-farnesol

La protéine recombinante Sh-SBS-6His a été incubée avec les substrats GPP, FPP et GGPP. Quel que soit le substrat utilisé, aucun nouveau terpène n'a pu être détecté, signifiant que l'enzyme est inactive sur les substrats trans-allyliques (tableau 1).

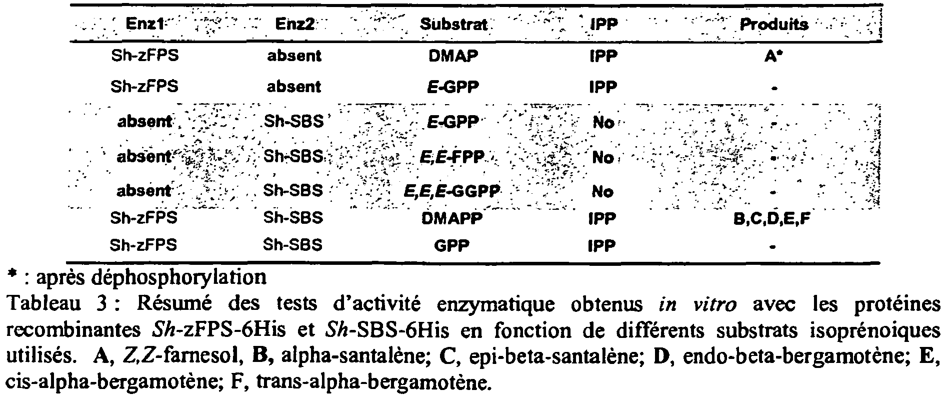

Les enzymes *Sh*-zFPS-6His et *Sh*-SBS-6His ont été incubées ensemble avec les substrats DMAPP et IPP ou GPP et IPP. Le produit de la réaction n'a pas été traité à la phosphatase, mais extrait avec du pentane dans lequel toute oléfine est soluble. Avec le DMAPP et l'IPP comme substrats, l'analyse chromatographique de l'extrait révèle la présence de plusieurs composés terpéniques (Fig. 5). Le profil GC a été comparé à celui de l'exsudat de la lignée de tomate TA517. Cette lignée est une lignée quasi-isogénique obtenue à partir des parents *S. lycopersicum* et *S*. *habrochaites* (Monforte and Tanksley, 2000). La lignée TA517 possède un fragment d'introgression de *S*. *habrochaites* responsable de la biosynthèse des sesquiterpènes de classe II (van der Hoeven *et al.*, 2000). Les deux profils GC sont identiques ce qui confirme que les enzymes recombinantes ont une activité strictement identique à celles présentes dans la tomate (Fig. 6). Le composé majoritaire (Fig. 5, pic 2) est identifié comme l'alpha-santalène par comparaison de son temps de rétention et son spectre de masse avec celui de l'extrait d'exsudat de tomate TA517. Par ordre de quantités décroissantes, l'alpha-santalène (pic 4), l'endo-beta-bergamotène (pic 5), et l'alpha-bergamotène (pic 1) ont également été identifiés. L'enzyme Sh-SBS-6His est donc une enzyme dont l'activité conduit à la formation de plusieurs produits.

Il peut être conclu que Sh-zFPS est une enzyme qui agit sur l'IPP et le DMAPP pour produire un composé phosphorylé de type farnesyl diphosphate. Le temps de rétention est identique au standard (*Z,Z*)-farnesol indiquant que le famesyldiphosphate produit par Sh-zFPS est caractérisé par une configuration de type *Z,Z*. L'inactivité de Sh-zFPS-6His sur le *E*-geranyl diphosphate en combinaison avec l'IPP indique que cette molécule n'est pas un produit intermédiaire de Sh-zFPS-6His et produit au moins une liaison de type *Z* en combinant un DMAPP et un IPP. Sh-SBS est actif uniquement sur le composé farnesyldiphosphate produit par l'enzyme Sh-zFPS, mais produit au moins 4 sesquiterpènes clairement identifiés dont le majoritaire est l'alpha-santalène. Enfin, la présence d'une extension en C-terminale par un *spacer* et 6 histidines, ne modifie en rien l'activité de l'enzyme par rapport aux enzymes natives de la plante d'origine (Fig. 3).

### Expression des gènes Z,Z-FPP synthase (Sh-zFPS) et la SB synthase (Sh-SBS) dans le tabac

Un promoteur de tabac spécifique des trichomes (eCBTS02, Tissier *et al.*, 2004) a été cloné en amont des séquences codantes *Sh-zFPS* et *Sh-SBS* pour former les constructions eCBTS1.0-Sh-*zFPS* [#1] et eCBTS1.0-*Sh-SBS* [#2]. Ces deux constructions ont été introduites dans un même vecteur binaire pour la transformation par *Agrobacterium,* contenant un gène de résistance à la kanamycine pour donner le vecteur binaire pLIBRO-064 (Fig. 2A). La construction #2 a également été introduite de manière indépendante dans un vecteur binaire pLIBRO-065 (Fig. 2B). Les vecteurs ont été introduits dans la souche d'*Agrobacterium* LBA4404 par électroporation. Les souches contenant les différents vecteurs ont été utilisées pour réaliser la transformation génétique de *N. sylvestris* par la méthode des disques foliaires (Horsch *et al.*, 1985).

Les T-DNA portant les transgènes ont été introduits par transformation génétique dans une lignée transgénique de *N. sylvestris* (lignée source ihpCBTS) dont l'expression du gène CBTS a été inhibée par ARN interférence (Tissier *et al.*, 2005). Dans cette lignée, la quantité de CBT-diol des feuilles est très réduite. *N. sylvestris*, un tabac sauvage ne produit pas de sesquiterpènes équivalents à ceux de la tomate. Environ 25 plantes résistantes à la kanamycine ont été obtenues pour chaque construction. La présence des transgènes dans chaque plante a été confirmée par PCR à partir d'ADN génomique de feuilles. Le niveau d'expression des transgènes dans les feuilles a été vérifié par PCR quantitative en temps réel à partir de mRNA de feuilles de tabac. Le niveau d'expression du transgène *Sh-SBS* a été comparé à celui d'un gène actine dont l'expression est constitutive dans les feuilles de tabac. Les résultats sont présentés sur la figure 3 en exemple. Ils indiquent que les lignées sélectionnées expriment toutes le transgène à des niveaux variables de 1 à 50 pour la construction pLIBRO-064 et de 1 à 10 pour la construction pLIBRO-065 par rapport au gène d'actine.

Les sesquiterpènes oléfiniques étant des molécules volatiles, les molécules volatiles émises par les plantes ont été analysées en atmosphère contrôlée dans une enceinte de culture. Une partie de l'atmosphère a été captée dans un circuit de dérivation contenant un filtre SuperQ® (Altech) ayant la capacité d'adsorber les molécules terpéniques oléfiniques. Les molécules ont ensuite été éluées avec 1 ml de pentane et analysées par GC/MS. Un exemple de profil chromatographique des molécules volatiles émises par une plante transgénique est présenté sur la figure 7. Il montre que la lignée #3877 exprimant les gènes *Sh-zFPS* et *Sh-SBS* contient plusieurs nouveaux pics possédant une signature aux ions moléculaires 93, 94, 121 et 204 caractéristiques des sesquiterpènes de cette classe. Le profil chromatographique a été comparé à celui obtenu à partir de l'exsudat de la lignée de tomate TA517 qui produit naturellement ces composés (Fig. 7). Les deux profils sont identiques et les spectres de masse de chacun des pics sont identiques à ceux des pics de la lignée TA517. Les plantes qui expriment les deux transgènes *Sh-zFPS* et *Sh-SBS* produisent de l'alpha-santalène, de l'épi-beta-santalène, du cis-alpha-bergamotène, du trans-alpha-bergamotène et de l'endo-beta-bergamotène tandis que les lignées transgéniques ayant intégré le gène *Sh-SBS* seul ne produisent aucun de ces composés. Ces résultats indiquent que la présence des deux gènes est nécessaire à la synthèse des sequiterpènes de type SB. Ils confirment les résultats obtenus *in vitro* avec les enzymes recombinantes. En conclusion, les gènes *Sh-zFPS* et *Sh-SBS* sont responsables de la biosynthèse des sesquiterpènes de tomate localisés au niveau du locus Sst2 décrit par van der Hoeven *et al.*, 2000.

### Polymorphisme génétique du gène zFPS entre S. lycopersicum et S. habrochaites.

Le gène complet ***zFPS*** a été amplifié par PCR à partir d'ADN génomique de *S*. ***lycopersicum,** S. habrochaites* et TA517 en utilisant les amorces ACCATGGGTTCTTTGGTTCTTCAATGTTGGA (SEQ ID No 9) et ACTCGAGATATGTGTGTCCACCAAAACGTCTATG (SEQ ID No 10). Deux produits ont été amplifiés dans les deux génomes indiquant qu'il existe au moins deux copies du gène dans les deux génomes (Fig. 8). L'un des deux produits qui possède une taille d'environ 3000 nucléotide (bande A) est commun aux deux génomes. Le second produit possède une taille d'environ 2000 nucléotides (bande C) chez *S*. *lycopersicum* et d'environ 2500 nucléotides (bande B) chez *S*. *habrochaites*. La lignée TA517 montre un profil identique à celui de *S*. *habrochaites*. Ces résultats indiquent que le gène B spécifique de *S*. *habrochaites* a été introduit dans la lignée TA517. Le séquençage du produit B a permis de confirmer qu'il correspond au gène ***zFPS*** présenté dans ce document. Ce résultat montre également que le gène ***zFPS*** de *S*. *habrochaites* peut être introduit dans *S*. *lycopersicum* en utilisant le polymorphisme qui existe entre les deux génomes pour ce gène.

### Références

Altschul, Stephen F., Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402.
Bates GW, Gaynor JJ, Shekhawat NS. (1983) Fusion of Plant Protoplasts by Electric Fields. Plant Physiol. 72:1110-1113..
Bohlmann J, Crock J, Jetter R, Croteau R. (1998) Terpenoid-based defenses in conifers: cDNA cloning, characterization, and functional expression of wound-inducible (E)-alpha-bisabolene synthase from grand fir (Abies grandis). Proc Natl Acad Sci U S A. 1998 95:6756-6761.
Bouwmeester HJ, Kodde J, Verstappen FW, Altug IG, de Kraker JW, Wallaart TE. (2002) Isolation and characterization of two germacrene A synthase cDNA clones from chicory. Plant Physiol. 29:134-144.
Cai Y, Jia JW, Crock J, Lin ZX, Chen XY, Croteau R. (2002) A cDNA clone for beta-caryophyllene synthase from Artemisia annua. Phytochemistry. 61:523-529.
Cane DE. (1999) Sesquiterpene biosynthesis: Cyclization mechanisms. In comprehensive natural products chemistry, isoprenoids including carotenoids and steroids, Vol. 2, D.D. Cane, ed (Amsterdam, The Netherlands;Elsever), pp.155-200.
Carman RM, Duffield AR (1993) The Biosynthesis of Labdanoids - the Optical Purity of Naturally-Occurring Manool and Abienol. Aust. J. Chem. 46 : 1105-1114.
Clarke CF, Tanaka RD, Svenson K, Wamsley M, Fogelman AM, Edwards PA (1987) Molecular cloning and sequence of a cholesterol-repressible enzyme related to prenyltransferase in the isoprene biosynthetic pathway. Mol. Cell. Biol 7: 3138-3146.
Colby SM, Crock J, Dowdle-Rizzo B, Lemaux PG, Croteau R. Germacrene C synthase from Lycopersicon esculentum cv. VFNT cherry tomato: cDNA isolation, characterization, and bacterial expression of the multiple product sesquiterpene cyclase (1998) Proc Natl Acad Sci 95:2216-2221.
Facchini PJ, Chappell J. Gene family for an elicitor-induced sesquiterpene cyclase in tobacco (1992) Proc Natl Acad Sci 89(22):11088-11092.
Heinstein PF, Herman DL, Tove SB, Smith FH. Biosynthesis of gossypol. Incorporation of mevalonate-2-14C and isoprenyl pyrophosphates (1970) J. Biol. Chem. 245: 4658-4665.
Horsch RB, Rogers SG, Fraley RT (1985) Transgenic plants. Cold Spring Harb Symp Quant Biol. 50: 433-437.
Kharel Y, Takahashi S, Yamashita S, Koyama T. (2006) Manipulation of prenyl chain length détermination mechanism of cis-prenyltransferases. FEBS J. 273:647-657.
Kollner TG, O'Maille PE, Gatto N, Boland W, Gershenzon J, Degenhardt J. (2006) Two pockets in the active site of maize sesquiterpene synthase TPS4 carry out sequential parts of the reaction scheme resulting in multiple products. Arch Biochem Biophys. 448:83-92.
Koyama T (1999) Molecular analysis of prenyl chain elongation enzymes. Biosci. Biotechnol. Biochem. 63:1671-1676.
Liang PH, Ko TP, Wang AH. (2002) Structure, mechanism and function of prenyltransferases. (2002) 269:3339-3354.
Marchler-Bauer A, Bryant SH (2004), "CD-Search: protein domain annotations on the fly.", Nucleic Acids Res.32(W)327-331.
McMillan J., and Beale M.H. (1999) Dipterpene biosynthesis. In comprehensive natural products chemistry, isoprenoids including carotenoids and steroids Vol. 2, D.D. Cane, ed (Amsterdam, The Netherlands;Elsever), pp.217-243.
Monforte AJ, Tanksley SD (2000) Development of a set of near isogenic and backcross recombinant inbred lines containing most of the Lycopersicon hirsutum genome in an L. esculentum background: A tool for gene mapping and gene discovery. Genomic 43:803-813.
Oh SK, Han KH, Ryu SB, Kang H. (2000) Molecular cloning, expression, and functional analysis of a cis-prenyltransferase from Arabidopsis thaliana. Implications in rubber biosynthesis. J Biol Chem. 275:18482-18488.
Poulter DC (2006) Farnesyl Diphosphate Synthase. A Paradigm for Understanding Structure and Function Relationships in E-polyprenyl Diphosphate Synthases. Phytochem review 5:17-26.
Rodriguez-Concepcion M, Boronat A. (2002) Elucidation of the methylerythritol phosphate pathway for isoprenoid biosynthesis in bacteria and plastids. A metabolic milestone achieved through genomics. Plant Physiol. 130(3):1079-89.
Sharon-Asa L, Shalit M, Frydman A, Bar E, Holland D, Or E, Lavi U, Lewinsohn E, Eyal Y. (2003) Citrus fruit flavor and aroma biosynthesis: isolation, functional characterization, and developmental regulation of Cstps1 a key gene in the production of the sesquiterpene aroma compound valencene. Plant J. 2003 36:664-74.
Shimizu N, Koyama T, Ogura K. (1998) Molecular cloning, expression, and purification of undecaprenyl diphosphate synthase. No sequence similarity between E- and Z-prenyl diphosphate synthases. J Biol Chem.273:19476-19481.
Schulbach MC, Brennan PJ, Crick DC. (2000) Identification of a short (C15) chain Z-isoprenyl diphosphate synthase and a homologous long (C50) chain isoprenyl diphosphate synthase in Mycobacterium tuberculosis. J Biol Chem. 275:22876-22881.
Tarshis LC, Yan M, Poulter CD, Sacchettini JC. (1994) Crystal structure of recombinant farnesyl diphosphate synthase at 2.6-A resolution. Biochemistry 33:10871-10877.
Tholl D. (2006) Terpene synthases and the regulation, diversity and biological roles ofterpene metabolism Curr Opin Plant Biol. 9:297-304.
Thompson,J.D., Gibson,T.J., Plewniak,F., Jeanmougin,F. and Higgins,D.G. (1997) The ClustalX windows interface: flexible strategies for multiple sequence alignment aided by quality analysis tools. Nucleic Acids Research 24:4876-4882.
Tissier A, Sallaud C, Rontein D (2004) Promoteurs végétaux et utilisations PCT/FR 05/02530.
Tissier A, Sallaud C, Rontein D (2005) Système de production de terpènes dans les plantes Demande de brevet France FR 05 00855.
van Der Hoeven RS, Monforte AJ, Breeden D, Tanksley SD, Steffens JC. (2000) Free in PMC Genetic control and evolution of sesquiterpene biosynthesis in Lycopersicon esculentum and L. hirsutum. Plant Cell. 12:2283-2294.
Wallaart TE, Bouwmeester HJ, Hille J, Poppinga L, Maijers NC (2001) Amorpha-4,11-diene synthase: cloning and functional expression of a key enzyme in the biosynthetic pathway of the novel antimalarial drug artemisinin. Planta. 212:460-465.
Wang K, Ohnuma SI (1999) Chain-length determination mechanism of isoprenyl diphosphate synthases and implications for molecular evolution 24 :445-451.
Wise ML, Croteau RA (1999). Monoterpene biosynthesis. In comprehensive natural products chemistry, isoprenoids including carotenoids and steroids Vol. 2, D.D. Cane, ed (Amsterdam, The Netherlands;Elsever), pp.97-153.
Zimmermann U, Scheurich P. (1981) High frequency fusion of plant protoplasts by electric fields. Planta 151:26-32

## Revendications

1. Méthode de production de sesquiterpènes de type SB (Santalène et Bergamotène) ou de dérivés de ceux-ci à partir de *Z,Z*-FPP (*Z,Z*-farnesyl diphosphate) dans une cellule ayant une source de DMAP et IPP, comprenant :
a) l'introduction dans ladite cellule d'une construction portant une cassette d'expression comprenant un premier gène codant pour une zFPS (*Z,Z*-farnesyl diphosphate synthase) présentant au moins 80 % d'identité avec la SEQ ID No 2 et d'une construction portant une cassette d'expression comprenant un deuxième gène codant pour une SB synthase présentant au moins 80 % d'identité avec la SEQ ID No 4;
b) la culture de la cellule transformée dans des conditions appropriées pour l'expression desdits premier et deuxième gènes ; et,
c) facultativement, la récolte de sesquiterpènes de type SB ou de dérivés de celui-ci contenus dans ladite cellule et/ou dans le milieu de culture.

2. Protéine isolée ou recombinante ayant une activité Z,Z-FPP synthase et une séquence présentant au moins 80% d'identité avec la SEQ ID No 2.

3. Protéine selon la revendication 2, **caractérisée en ce que** sa séquence correspond à la SEQ ID No 2.

4. Protéine isolée ou recombinante ayant une activité de SB synthase et ayant une séquence présentant au moins 80 % d'identité avec la SEQ ID No 4.

5. Cassette d'expression ou vecteur comprenant une séquence nucléotidique hétérologue codant pour une protéine selon les revendications 2 à 4.

6. Cellule hôte comprenant un acide nucléique, une cassette d'expression ou un vecteur comprenant une séquence nucléotidique hétérologue codant pour une protéine selon les revendications 2 à 4..

7. Organisme transgénique non-humain comprenant un acide nucléique, une cassette d'expression ou un vecteur comprenant une séquence nucléotidique hétérologue codant pour une protéine selon les revendications 2 à 4, ou une cellule selon la revendication 6.

8. Organisme transgénique selon la revendication 7, **caractérisé en ce que** l'organisme est une plante à trichomes glanduleux, de préférence du genre Nicotiana ou Solanum.

9. Méthode de production du *Z,Z*-farnesyl diphosphate (*Z,Z*-FPP), ou de dérivés de ceux-ci à partir d'IPP et de DMAP dans une cellule ayant une source d'IPP et de DMAP, comprenant :
a) l'introduction dans ladite cellule d'une construction portant une cassette d'expression avec une séquence nucléique codant une protéine selon la revendication 2 ou 3 ;
b) la culture de la cellule transformée dans des conditions appropriées pour l'expression du gène ; et
c) facultativement, la récolte du Z,Z-FPP ou de dérivés de celui-ci contenus dans ladite cellule et/ou dans le milieu de culture.

10. Utilisation d'une protéine selon les revendications 2 à 4, d'un acide nucléique selon la revendication 5, d'une cellule hôte selon la revendication 6 ou d'un organisme transgénique selon la revendication 7 ou 8 pour la préparation de *Z,Z*-FPP, d'alpha-santalène, d'épi-beta-santalène, d'alpha-bergamotène, de beta-bergamotène, et d'endo-beta-bergamotène ou de dérivés de ceux-ci comme l'alpha-santalol, l'épi-beta-santalol, l'alpha-bergamotol, le beta-bergamotol et le *Z,Z*-farnesol.

11. Cellule ou organisme transgénique non-humain, **caractérisé en ce que** la voie de synthèse de sesquiterpène de type SB est bloquée par inactivation du gène codant pour une protéine selon la revendication 2 ou 3, ou du gène codant pour une protéine selon la revendication 4 ou des deux gènes.

12. Utilisation de marqueurs moléculaires comprenant tout ou partie d'un acide nucléique présentant au moins 80 % d'identité avec la SEQ ID No 1 ou SEQ ID No 3 pour identifier un polymorphisme génomique entre S. habrochaites et des espèces de type Solanum sexuellement compatible avec S. habrochaites afin de contrôler l'introgression des gènes correspondant dans ces espèces.

13. Utilisation selon la revendication 12, **caractérisée en ce que** les marqueurs moléculaires comprennent des séquences nucléiques correspondant aux SEQ ID No. 9 et 10.

14. Méthode consistant à introduire le gène *zFPS* codant pour une zFPS (*Z,Z*-farnesyl diphosphate synthase) présentant au moins 80 % d'identité avec la SEQ ID No 2 et le gène *SBS* (SB synthase) codant pour une SB (Santalène et Bergamotène) synthase présentant au moins 80 % d'identité avec la SEQ ID No 4 dans une espèce non sexuellement compatible avec *Solanum habrochaites* par fusion de protoplastes.

15. Méthode selon la revendication 9, comprenant en outre la transformation du Z,Z-FPP obtenu en Z,Z-farnesol par une phosphatase.

16. Méthode de production de sesquiterpènes de type SB (Santalène et Bergamotène) ou de dérivés de ceux-ci à partir de *Z,Z*-FPP (*Z,Z*-farnesyl diphosphate) dans une cellule ayant une source de DMAP et IPPP, comprenant :
a) la fourniture d'une cellule recombinante comprenant un premier gène hétérologue codant pour une zFPS (*Z,Z*-farnesyl diphosphate synthase) présentant au moins 80 % d'identité avec la SEQ ID No 2 et un deuxième gène hétérologue codant pour une SB synthase présentant au moins 80 % d'identité avec la SEQ ID No 4;
b) la culture de la cellule dans des conditions appropriées pour l'expression desdits premier et deuxième gènes ; et,
c) facultativement, la récolte de sesquiterpènes de type SB ou de dérivés de celui-ci contenus dans ladite cellule et/ou dans le milieu de culture.

17. Méthode de production de *Z,Z*-FPP dans une cellule recombinante ayant une source de DMAP et IPPP, comprenant :
a) la fourniture d'une cellule recombinante comprenant un gène hétérologue codant pour une zFPS présentant au moins 80 % d'identité avec la SEQ ID No 2 ;
b) la culture de la cellule dans des conditions appropriées pour l'expression dudit gène ; et,
c) facultativement, la récolte du *Z,Z*-FPP ou de dérivés de celui-ci contenus dans ladite cellule et/ou dans le milieu de culture.

18. Méthode de production de sesquiterpènes de type SB ou de dérivés de ceux-ci à partir de *Z,Z*-FPP dans une cellule recombinante ayant une source de *Z,Z*-FPP, comprenant :
a) la fourniture d'une cellule recombinante comprenant un gène hétérologue codant pour une SB synthase présentant au moins 80 % d'identité avec la SEQ ID No 4;
b) la culture de la cellule dans des conditions appropriées pour l'expression dudit gène ; et,
c) facultativement, la récolte de sesquiterpènes de type SB ou de dérivés de celui-ci contenus dans ladite cellule et/ou dans le milieu de culture.

## Patentansprüche

1. Verfahren zur Herstellung von Sesquiterpenen des SB-Typs (Santalen und Bergamoten) oder von Derivaten davon, ausgehend von Z,Z-FPP (Z,Z-Farnesyldiphosphat) in einer Zelle mit einer DMAP- und IPP-Quelle, umfassend:
a) die Einführung in besagte Zelle eines Konstrukts, das eine Expressionskassette trägt, umfassend ein erstes Gen, das für eine zFPS (Z,Z-Farnesyldiphosphat-Synthase) codiert, die wenigstens zu 80% mit der SEQ ID NR: 2 identisch ist, und eines Konstrukts, das eine Expressionskassette trägt, umfassend ein zweites Gen, das für eine SB-Synthase codiert, die wenigstens zu 80% mit der SEQ ID NR: 4 identisch ist;
b) die Kultivierung der transformierten Zelle unter Bedingungen, die für die Expression besagten ersten und zweiten Gens geeignet sind; und
c) fakultativ die Gewinnung von Sesquiterpenen des SB-Typs oder von Derivaten davon, die in besagter Zelle und/oder im Kulturmedium enthalten sind.

2. Rekombinantes oder isoliertes Protein mit einer Z,Z-FPP-Synthaseaktivität und einer Sequenz, die wenigstens zu 80% mit der SEQ ID NR: 2 identisch ist.

3. Protein gemäß Anspruch 2, **dadurch gekennzeichnet, dass** seine Sequenz der SEQ ID NR: 2 entspricht.

4. Rekombinantes oder isoliertes Protein mit einer SB-Synthaseaktivität und mit einer Sequenz, die wenigstens zu 80% mit der SEQ ID NR: 4 identisch ist.

5. Expressionskassette oder Vektor, umfassend eine heterologe Nukleotidsequenz, die für ein Protein gemäß Ansprüchen 2 bis 4 codiert.

6. Wirtszelle, umfassend eine Nukleinsäure, eine Expressionskassette oder einen Vektor, umfassend eine heterologe Nukleotidsequenz, die für ein Protein gemäß Ansprüchen 2 bis 4 codiert.

7. Nicht-humaner transgener Organismus, umfassend eine Nukleinsäure, eine Expressionskassette oder einen Vektor, umfassend eine heterologe Nukleotidsequenz, die für ein Protein gemäß Ansprüchen 2 bis 4 codiert, oder eine Zelle gemäß Anspruch 6.

8. Transgener Organismus gemäß Anspruch 7, **dadurch gekennzeichnet, dass** der Organismus eine Pflanze mit Drüsenhaaren vorzugsweise der Gattung Nicotiana oder Solanum ist.

9. Verfahren zur Herstellung von Z,Z-Farnesyldiphosphat (Z,Z-FPP) oder von Derivaten davon, ausgehend von IPP oder DMAP in einer Zelle mit einer DMAP- und IPP-Quelle, umfassend:
a) die Einführung in besagte Zelle eines Konstrukts, das eine Expressionskassette mit einer Nukleinsäuresequenz trägt, die ein Protein gemäß Anspruch 2 oder 3 codiert;
b) die Kultivierung der transformierten Zelle unter Bedingungen, die für die Expression des Gens geeignet sind; und
c) fakultativ die Gewinnung des Derivates von Z,Z-FPP oder der Derivate davon, die in besagter Zelle und/oder im Kulturmedium enthalten sind.

10. Verwendung eines Proteins gemäß Ansprüchen 2 bis 4, einer Nukleinsäure gemäß Anspruch 5, einer Wirtszelle gemäß Anspruch 6 oder eines transgenen Organismus gemäß Anspruch 7 oder 8 zur Darstellung von Z,Z-FPP, alpha-Santalen, epi-beta-Santalen, alpha-Bergamoten, beta-Bergamoten und endo-beta-Bergamoten oder Derivaten davon, wie alpha-Santalol, epi-beta-Santalol, alpha-Bergamotol, beta-Bergamotol und Z,Z-Farnesol.

11. Zelle oder nicht-humaner transgener Organisums, **dadurch gekennzeichnet, dass** der Syntheseweg von Sesquiterpen des SB-Typs durch eine Inaktivierung des Gens, das für ein Protein gemäß Anspruch 2 oder 3 codiert, oder des Gens, das für ein Protein gemäß Anspruch 4 codiert, oder der beiden Gene blockiert ist.

12. Verwendung von molekularen Markern, umfassend die gesamte oder einen Abschnitt der Nukleinsäure, die wenigstens zu 80% mit der SEQ ID NR: 1 oder SEQ ID NR: 3 identisch ist, zur Identifizierung eines genomischen Polymorphismus von S. habrochaites und Solanum-Spezies, die mit S. habrochaites sexuell kompatibel sind, um die Introgression der korrespondierenden Gene in diesen Spezies zu kontrollieren.

13. Verwendung gemäß Anspruch 12, **dadurch gekennzeichnet, dass** die molekularen Marker Sequenzen umfassen, die SEQ ID NR: 9 und 10 entsprechen.

14. Verfahren, bestehend aus Einführung des zFPS-Gens, das für eine zFPS (Z,Z-Farnesyldiphosphat-Synthase) codiert, die wenigstens zu 80% mit SEQ ID NR: 2 identisch ist, und des SBS-(SB-Synthase)-Gens, das für eine SB-(Santalen und Bergamoten)-Synthase codiert, die wenigstens zu 80% mit SEQ ID NR: 4 identisch ist, in eine mit Solanum habrochaites nicht sexuell kompatible Spezies mittels Protoplastenfusion.

15. Verfahren gemäß Anspruch 9, umfassend außerdem die Umwandlung des erhaltenen Z,Z-FPP in Z,Z-Farnesol mithilfe einer Phosphatase.

16. Verfahren zur Herstellung von Sesquiterpenen des SB-Typs (Santalen und Bergamoten) oder von Derivaten davon, ausgehend von Z,Z-FPP (Z,Z-Farnesyldiphosphat) in einer Zelle mit einer DMAP- und IPPP-Quelle, umfassend:
a) die Bereitstellung einer rekombinanten Zelle, umfassend ein erstes heterologes Gen, das für eine zFPS (Z,Z-Farnesyldiphosphat-Synthase) codiert, die wenigstens zu 80% mit der SEQ ID NR: 2 identisch ist, und ein zweites heterologes Gen, das für eine SB-Synthase codiert, die wenigstens zu 80% mit der SEQ ID NR: 4 identisch ist;
b) die Kultivierung der Zelle unter Bedingungen, die für die Expression besagten ersten und zweiten Gens geeignet sind; und
c) fakultativ die Gewinnung von Sesquiterpenen des SB-Typs oder von Derivaten davon, die in besagter Zelle und/oder im Kulturmedium enthalten sind.

17. Verfahren zur Herstellung von Z,Z-FPP in einer rekombinanten Zelle mit einer DMAP- und IPPP-Quelle, umfassend:
a) die Bereitstellung einer rekombinanten Zelle, umfassend ein heterologes Gen, das für eine zFPS codiert, die wenigstens zu 80% mit der SEQ ID NR: 2 identisch ist,
b) die Kultivierung der Zelle unter Bedingungen, die für die Expression besagten Gens geeignet sind; und
c) fakultativ die Gewinnung von Z,Z-FPP oder von Derivaten davon, die in besagter Zelle und/oder im Kulturmedium enthalten sind.

18. Verfahren zur Herstellung von Sesquiterpenen des SB-Typs oder von Derivaten davon, ausgehend von Z,Z-FPP in einer rekombinanten Zelle mit einer Z,Z-FPP-Quelle, umfassend:
a) die Bereitstellung einer rekombinanten Zelle, umfassend ein heterologes Gen, das für eine SB-Synthase codiert, die wenigstens zu 80% mit der SEQ ID NR: 4 identisch ist,
b) die Kultivierung der Zelle unter Bedingungen, die für die Expression besagten Gens geeignet sind; und
c) fakultativ die Gewinnung von Sesquiterpenen des SB-Typs oder von Derivaten davon, die in besagter Zelle und/oder im Kulturmedium enthalten sind.

## Claims

1. A method for producing SB (Santalene and Bergamotene) type sesquiterpenes or derivatives thereof from Z,Z-FPP (Z,Z-farnesyl diphosphate) in a cell having a source of DMAP and IPP, comprising:
a) introducing into said cell a construct having an expression cassette comprising a first gene encoding a zFPS (Z,Z-farnesyl diphosphate synthase) with at least 80% identity to SEQ ID No. 2 and a construct having an expression cassette comprising a second gene encoding a SB synthase with at least 80 % identity to SEQ ID No. 4;
b) culturing the transformed cell in suitable conditions for the expression of said first and said second genes; and,
c) optionally, collecting SB type sesquiterpenes or derivatives thereof contained in said cell and/or in the culture medium.

2. An isolated or recombinant protein having Z,Z-FPP synthase activity and a sequence with at least 80% identity to SEQ ID No. 2.

3. The protein according to claim 2, **characterized in that** its sequence corresponds to SEQ ID No 2.

4. An isolated or recombinant protein having SB synthase activity and having a sequence with at least 80% identity to SEQ ID No. 4.

5. An expression cassette or a vector comprising a heterologous nucleotide sequence encoding a protein according to claims 2 to 4.

6. A host cell comprising a nucleic acid, an expression cassette or a vector comprising a heterologous nucleotide sequence encoding a protein according to claims 2 to 4.

7. A non-human transgenic organism comprising a nucleic acid, an expression cassette or a vector comprising a heterologous nucleotide sequence encoding a protein according to claims 2 to 4, or a cell according to claim 6.

8. The transgenic organism according to claim 7, **characterized in that** the organism is a plant with glandular trichomes, preferably from the genus Nicotiana or Solanum.

9. A method for producing *Z,Z*-farnesyl diphosphate (*Z,Z*-FPP), or derivatives thereof from IPP and DMAP in a cell having an IPP and DMAP source, comprising:
a) introducing into said cell a construct having an expression cassette with a nucleic sequence encoding a protein according to claim 2 or 3;
b) culturing the transformed cell in suitable conditions for the expression of the gene; and
c) optionally, collecting Z,Z-FPP or the derivative(s) thereof contained in said cell and/or in the culture medium.

10. Use of a protein according to claims 2 to 4, of a nucleic acid according to claim 5, of a host cell according to claim 6 or of a transgenic organism according to claim 7 or 8 for preparing *Z,Z*-FPP, alpha-santalene, epi-beta-santalene, alpha-bergamotene, beta-bergamotene, and endo-beta-bergamotene or derivatives thereof such as alpha-santalol, epi-beta-santalol, alpha-bergamotol, beta-bergamotol and *Z,Z*-farnesol.

11. A cell or non-human transgenic organism, **characterized in that** the synthetic pathway of SB type sesquiterpene is blocked by inactivation of the gene encoding a protein according to claim 2 or 3, or of the gene encoding a protein according to claim 4, or both genes.

12. Use of molecular markers comprising all or part of a nucleic acid with at least 80 % identity to SEQ ID No. 1 or SEQ ID No. 3 for identifying a genomic polymorphism between *S. habrochaites* and species of the *Solanum* type that are sexually compatible with S. *habrochaites* so as to control the introgression of the corresponding genes in said species.

13. Use according to claim 12, **characterized in that** the molecular markers comprise the nucleic sequences corresponding to SEQ ID No. 9 and 10.

14. A method consisting in introducing the *zFPS* gene encoding a zFPS (Z,Z-farnesyl diphosphate synthase) with at least 80 % identity to SEQ ID No. 2 and the *SBS* (SB synthase) gene encoding a SB (Santalene and Bergamotene) synthase with at least 80 % identity to SEQ ID No. 4 in a species not sexually compatible with *Solanum habrochaites* by protoplast fusion.

15. Method according to claim 9, comprising the conversion of Z,Z-FPP into Z,Z-farnesol by a phosphatase.

16. Method for producing SB (Santalene and Bergamotene) type sesquiterpenes or derivatives thereof from Z,Z-FPP (Z,Z-farnesyl diphosphate) in a cell having a source of DMAP and IPPP, comprising:
a) providing a recombinant cell comprising a first heterologous gene encoding a zFPS (Z,Z-farnesyl diphosphate synthase) with at least 80 % identity to SEQ ID No. 2 and a second heterologous gene encoding a SB synthase with at least 80 % identity to SEQ ID No. 4;
b) culturing said cell in suitable conditions for the expression of said first and said second genes; and,
c) optionally, collecting the SB type sesquiterpenes or derivatives thereof contained in said cell and/or in the culture medium.

17. Method for producing *Z,Z*-FPP in a cell having a source of DMAP and IPPP, comprising:
a) providing a recombinant cell comprising a heterologous gene encoding a zFPS with at least 80 % identity to SEQ ID No. 2;
b) culturing said cell in suitable conditions for the expression of said first and said second genes; and,
c) optionally, collecting *Z,Z*-FPP or derivatives thereof contained in said cell and/or in the culture medium.

18. Method for producing SB type sesquiterpenes or derivatives thereof from *Z,Z*-FPP in a cell having a source of *Z,Z*-FPP, comprising:
a) providing a recombinant cell comprising a heterologous gene encoding a SB synthase with at least 80 % identity to SEQ ID No. 4;
b) culturing said cell in suitable conditions for the expression of said first and said second genes; and,
c) optionally, collecting the SB type sesquiterpenes or derivatives thereof contained in said cell and/or in the culture medium.
